(19) Europäisches Patentamt
European Patent Office
Office européen des brevets

(11) **EP 4 495 900 A1**

(12) **EUROPEAN PATENT APPLICATION**
published in accordance with Art. 153(4) EPC

(43) Date of publication:
**22.01.2025 Bulletin 2025/04**

(21) Application number: **23770208.9**

(22) Date of filing: **14.02.2023**

(51) International Patent Classification (IPC):
**G06V 40/40** *(2022.01)* **A61B 5/1171** *(2016.01)*

(52) Cooperative Patent Classification (CPC):
**A61B 5/1171; G06V 40/40**

(86) International application number:
**PCT/JP2023/004880**

(87) International publication number:
**WO 2023/176247 (21.09.2023 Gazette 2023/38)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **18.03.2022 JP 2022043843**

(71) Applicant: **Sony Group Corporation
Tokyo 108-0075 (JP)**

(72) Inventors:
• **TAKAHASHI, Ai
Tokyo 108-0075 (JP)**
• **SAKUMOTO, Koichi
Tokyo 108-0075 (JP)**

(74) Representative: **MFG Patentanwälte
Meyer-Wildhagen Meggle-Freund
Gerhard PartG mbB
Amalienstraße 62
80799 München (DE)**

(54) **INFORMATION PROCESSING DEVICE, INFORMATION PROCESSING SYSTEM, INFORMATION PROCESSING METHOD, AND PROGRAM**

(57)     An information processing apparatus (100) includes a control unit (130). The information processing apparatus (100) performs non-contact authentication of a living body using an image. The control unit (130) acquires a phase difference image acquired by an image plane phase difference sensor (200). The control unit (130) detects a background from the phase difference image. The control unit (130) determines whether or not impersonation has occurred on the basis of a focus position of the image plane phase difference sensor (200) in at least a part of the background.

FIG.3

EP 4 495 900 A1

**Description**

Field

The present disclosure relates to an information processing apparatus, an information processing system, an information processing method, and a program.

Background

With the development of information processing technology, biometric authentication, which is personal authentication using biometric information that is information unique to a living body, has been performed. As biometric authentication, for example, there is known an authentication system that collates a biometric image captured by a camera with a biometric image of the person in question acquired in advance to perform identity confirmation.

In the biometric authentication, there is a problem that a person other than the person in question impersonates the person in question and is illegally authenticated. In the non-contact biometric authentication using a camera image, there is a problem that it is possible to easily impersonate the person in question by displaying a photograph of the living body on paper or a display.

For example, there is known a technique for preventing the above-described impersonation by collating three-dimensional face information stored in advance in an apparatus with the face of an authentication target person on the basis of two-dimensional image data captured by a camera and image plane phase difference information (see, for example, Patent Literature 1).

Citation List

Patent Literature

Patent Literature 1: JP 2018-173731 A

Summary

Technical Problem

In the above-described technique, the three-dimensional face information of the authentication target person is generated using the two-dimensional image data and the image plane phase difference information. However, the depth information calculated using the image plane phase difference information has a problem of low accuracy.

Therefore, the present disclosure provides a mechanism capable of further improving authentication accuracy in non-contact authentication using biometric information.

Note that the above problem or object is merely one of a plurality of problems or objects that can be solved or achieved by a plurality of embodiments disclosed in the present specification. Solution to Problem

An information processing apparatus includes a control unit. The information processing apparatus performs non-contact authentication of a living body using an image. The control unit acquires a phase difference image acquired by an image plane phase difference sensor. The control unit detects a background from the phase difference image. The control unit determines whether or not impersonation has occurred on the basis of a focus position of the image plane phase difference sensor in at least a part of the background.

Brief Description of Drawings

FIG. 1 is a diagram for describing an outline of biometric authentication processing according to a first embodiment of the present disclosure.
FIG. 2 is a diagram for describing an example of impersonation.
FIG. 3 is a diagram for describing an outline of biometric authentication processing according to the first embodiment of the present disclosure.
FIG. 4 is a flowchart illustrating an example of a flow of biometric authentication processing according to the first embodiment of the present disclosure.
FIG. 5 is a diagram for describing an example of a camera.
FIG. 6 is a diagram illustrating an example of a captured image captured by a camera.

FIG. 7 is a diagram for describing an example of an image plane phase difference sensor according to the first embodiment of the present disclosure.

FIG. 8 is a diagram illustrating an example of a phase difference image captured by the image plane phase difference sensor.

FIG. 9 is a diagram for describing left and right images in a case where an object is at a focus position.

FIG. 10 is a diagram for describing left and right images in a case where an object is farther with respect to a focus position.

FIG. 11 is a diagram for describing left and right images in a case where an object is closer with respect to a focus position.

FIG. 12 is a block diagram illustrating a configuration example of an information processing system according to the first embodiment of the present disclosure.

FIG. 13 is a flowchart illustrating a flow of an example of the biometric authentication processing according to the first embodiment of the present disclosure.

FIG. 14 is a flowchart illustrating a flow of another example of the biometric authentication processing according to the first embodiment of the present disclosure.

FIG. 15 is a flowchart illustrating a flow of an example of guidance processing according to the first embodiment of the present disclosure.

FIG. 16 is a diagram illustrating an example of guidance information according to the first embodiment of the present disclosure.

FIG. 17 is a diagram illustrating another example of the guidance information according to the first embodiment of the present disclosure.

FIG. 18 is a diagram illustrating another example of the guidance information according to the first embodiment of the present disclosure.

FIG. 19 is a flowchart illustrating an example of impersonation determination processing according to the first embodiment of the present disclosure.

FIG. 20 is a diagram for describing an example of right patches according to the first embodiment of the present disclosure.

FIG. 21 is a diagram for describing an example of a left patch group according to the first embodiment of the present disclosure.

FIG. 22 is a flowchart illustrating an example of impersonation determination processing according to a second embodiment of the present disclosure.

FIG. 23 is a flowchart illustrating an example of impersonation determination processing according to a third embodiment of the present disclosure.

FIG. 24 is a flowchart illustrating an example of impersonation determination processing according to a fourth embodiment of the present disclosure.

FIG. 25 is a diagram illustrating an example of right patches for which the information processing apparatus according to the fourth embodiment of the present disclosure executes pattern matching.

FIG. 26 is a flowchart illustrating an example of impersonation determination processing according to a fifth embodiment of the present disclosure.

FIG. 27 is a flowchart illustrating an example of impersonation determination processing according to a sixth embodiment of the present disclosure.

FIG. 28 is a flowchart illustrating an example of a flow of guidance processing according to a seventh embodiment of the present disclosure.

FIG. 29 is a flowchart illustrating an example of a flow of impersonation determination processing according to the seventh embodiment of the present disclosure.

FIG. 30 is a block diagram illustrating an example of a hardware configuration of the information processing apparatus according to the present embodiment.

Description of Embodiments

Embodiments of the present disclosure will be described in detail below with reference to the accompanying drawings. Note that, in this specification and the drawings, components having substantially the same functional configuration are denoted by the same reference numerals, and redundant description is omitted.

One or a plurality of embodiments (including examples and modifications) described below can each be implemented independently. On the other hand, at least some of the plurality of embodiments described below may be implemented by being appropriately combined with at least some of other embodiments. The plurality of embodiments may include novel features different from each other. Accordingly, the plurality of embodiments can contribute to solving different objects or problems, and can exhibit different effects.

<<1. First Embodiment>>

<1.1. Outline>

Before describing a configuration of an information processing system according to the present embodiment, an outline of an information processing method according to the present embodiment will be described. In addition, in the following description, it is assumed that the information processing system according to the present embodiment performs processing related to the information processing method according to the present embodiment.

As described above, in the biometric authentication, the information processing system needs to prevent an attack (hereinafter, also simply referred to as "impersonation") through unauthorized access in which a person other than the person in question impersonates the person in question in some way and is illegally authenticated.

For example, a method in which a photograph of a living body is displayed on paper or a display to impersonate the person in question is easy for an attacker to prepare, and thus is easily used as a means for unauthorized access. As means for preventing the attack by the method, for example, means for detecting paper or a display before executing authentication is conceivable. For example, the information processing system can determine whether or not a biometric image used for authentication is impersonation by a photograph or the like by measuring three-dimensional information of a living body.

However, for example, in a normal RGB camera mounted in an information processing system such as a smartphone, it is difficult to perform three-dimensional measurement of a living body, which is an authentication target. In order to perform the three-dimensional measurement, the information processing system needs to include a sensor for performing three-dimensional measurement, such as a distance measuring sensor.

In general, in an information processing system such as a smartphone, an image plane phase difference sensor may be mounted as a part of an RGB camera in order to perform autofocus. By using this image plane phase difference sensor, the information processing system can perform three-dimensional measurement of a living body.

For example, the information processing system can measure the depth of the captured image from the left and right images generated by the image plane phase difference sensor, but there is a problem that the accuracy of depth measurement is low. In addition, the depth measurement using the image plane phase difference sensor has a problem that a calculation amount is large.

For example, in a case where the depth is calculated using the image plane phase difference sensor, the information processing system detects the parallax in each pixel of the left and right images acquired from the image plane phase difference sensor, and calculates the depth from the parallax. However, since the parallax of each pixel is small, it is difficult to detect with high accuracy, and it is difficult for the information processing apparatus to perform depth calculation with high accuracy.

In addition, the information processing system detects parallax for each pixel and calculates a depth. Therefore, when the depth is calculated in all the pixels, the calculation amount of the depth measurement increases.

As described above, in the method of performing the biometric authentication using the three-dimensional information detected using the image plane phase difference sensor mounted in the information processing system, the accuracy of the three-dimensional information is low, and the calculation time required for detecting the three-dimensional information is long. Therefore, it is difficult for the information processing system to perform impersonation determination with high accuracy and high calculation efficiency using the above method.

In the first embodiment of the present disclosure, the information processing system performs non-contact authentication of a living body using a captured image. The information processing system includes an image plane phase difference sensor, and acquires a phase difference image (for example, left and right images) acquired by the image plane phase difference sensor. The information processing system detects a background from the phase difference image. The information processing system determines whether or not impersonation has occurred on the basis of a comparison with a focus position of the image plane phase difference sensor in at least a part of the background. For example, the information processing system determines that impersonation has occurred when determining that at least a part of the background is the same as the focus position of the image plane phase difference sensor or is closer to the image plane phase difference sensor with respect to the focus position.

That is, the information processing system of the present disclosure does not detect the parallax of all the pixels of the left and right images, but determines impersonation on the basis of whether or not the living body and the background are on the same plane.

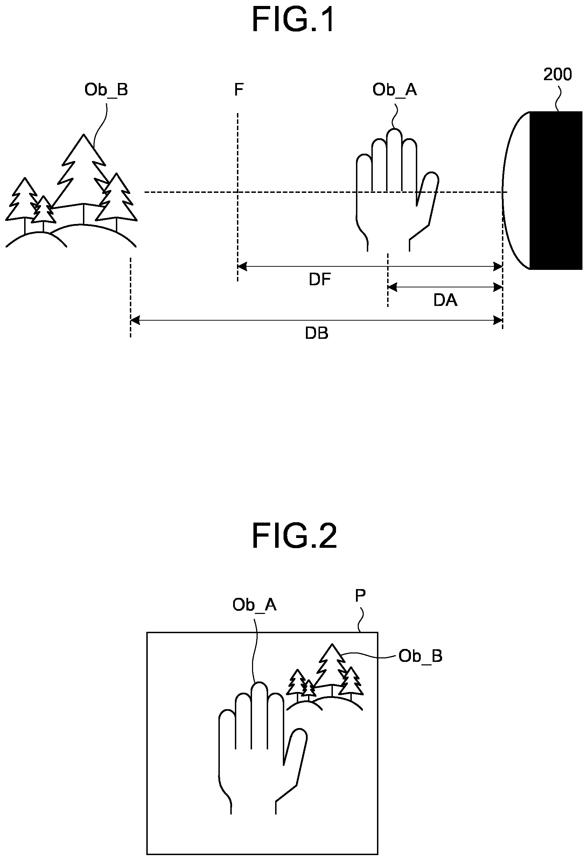

FIG. 1 is a diagram for describing an outline of biometric authentication processing according to the first embodiment of the present disclosure. Here, it is assumed that the information processing system performs authentication using a palm as a living body.

As illustrated in FIG. 1, it is assumed that a user performs authentication by holding a palm Ob_A in front of an image plane phase difference sensor 200 in front of a background Ob_B. In this case, the image plane phase difference sensor 200 generates a phase difference image including the palm Ob_A, which is an authentication target, and the background Ob_B.

Here, it is assumed that the palm Ob_A of the user is closer to the image plane phase difference sensor 200 with respect to a focus position F of the image plane phase difference sensor 200. That is, a distance DA between the image plane phase difference sensor 200 and the palm Ob_A is closer than a distance DF between the image plane phase difference sensor 200 and the focus position F (DA < DF).

On the other hand, the background Ob_B is farther from the image plane phase difference sensor 200 with respect to the focus position F of the image plane phase difference sensor 200. That is, a distance DB between the image plane phase difference sensor 200 and the background Ob_B is farther than the distance DF between the image plane phase difference sensor 200 and the focus position F (DB > DF).

As described above, when the person in question holds the palm Ob_A to perform the biometric authentication, the background Ob_B is located at a position farther from the focus position F.

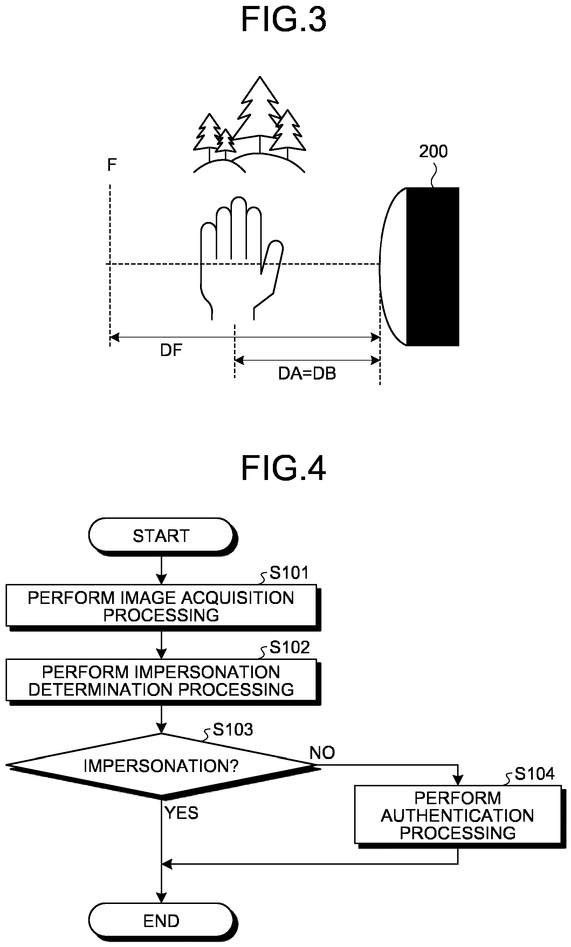

FIG. 2 is a diagram for describing an example of impersonation. FIG. 3 is a diagram for describing an outline of biometric authentication processing according to the first embodiment of the present disclosure. For example, it is assumed that a third party other than the person in question performs biometric authentication by impersonation. At this time, for example, as illustrated in FIG. 2, the third party performs the biometric authentication by impersonation by holding paper P on which the palm Ob_A of the user in question and the background Ob_B are printed over the image plane phase difference sensor 200.

In this case, since the palm Ob A and the background Ob_B are printed on the paper P, the palm Ob_A and the background Ob_B exist on the same plane. That is, the distance DB between the image plane phase difference sensor 200 and the background Ob_B is the same as the distance DA between the image plane phase difference sensor 200 and the palm Ob_A (DB = DA).

That is, the distance DB between the image plane phase difference sensor 200 and the background Ob_B is closer than the distance DF between the image plane phase difference sensor 200 and the focus position F (DB = DA < DF) similarly to the distance DA.

The information processing system determines whether or not the background Ob_B is closer with respect to the focus position F from the phase difference image acquired by the image plane phase difference sensor 200. In a case where the background Ob_B is closer with respect to the focus position F, the information processing system determines that impersonation has occurred.

FIG. 4 is a flowchart illustrating an example of a flow of biometric authentication processing according to the first embodiment of the present disclosure. The biometric authentication processing in FIG. 4 is performed by the information processing system.

As illustrated in FIG. 4, the information processing system executes image acquisition processing (Step S101). The information processing system acquires the phase difference image by executing the image acquisition processing.

Next, the information processing system executes impersonation determination processing (Step S102). For example, the information processing system determines whether or not impersonation has occurred by determining whether or not at least a part of the background included in the phase difference image is closer to the image plane phase difference sensor 200 with respect to the focus position F.

The information processing system determines whether or not a result of the impersonation determination processing is impersonation (Step S103). For example, the information processing system determines that impersonation has occurred in a case where at least a part of the background included in the phase difference image is closer to the image plane phase difference sensor 200 with respect to the focus position F.

When the information processing system determines that impersonation has occurred (Step S103; Yes), the information processing system ends the biometric authentication processing.

When the information processing system determines that impersonation has not occurred (Step S103; No), the information processing system performs authentication processing (Step S104).

As described above, the information processing system executes the impersonation determination processing before the authentication processing, so that the information processing system can prevent unauthorized access by a third party. In addition, the information processing system determine impersonation in accordance with the positional relationship between the background Ob_B and the focus position F. As a result, the information processing system can determine impersonation with higher accuracy.

The processing of determining whether or not the background Ob_B is closer with respect to the focus position F has a smaller calculation amount than the processing of calculating the depth for each pixel. Therefore, the information processing system can further reduce the calculation amount of the impersonation determination processing. The information processing system can determine impersonation in a shorter time.

Note that, here, the living body, which is an authentication target, is the palm of the user in question, but the living body, which is an authentication target, is not limited thereto. It is sufficient if the living body can be authenticated in a non-contact manner.

For example, in addition to the palm, for example, biometric information such as a finger (fingerprints, veins, joints, or the like), a face, and a gait can be an authentication target. For example, biometric information such as an ear (auricle) and an eye (iris, area around eyes, or the like) can be an authentication target. Note that, in a case where an ear (auricle), an eye (iris, area around eyes, or the like), or the like is a target, it is desirable that a background other than the living body (for example, the head or face) including the target be included in the phase difference image. Note that, in the following description, in order to simplify the description, it is assumed that the living body, which is an authentication target, is a palm.

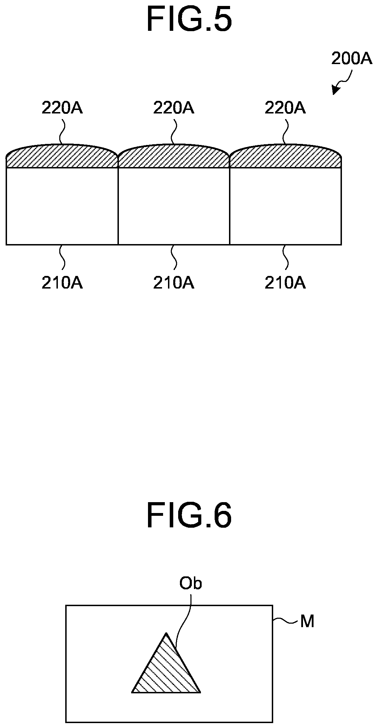

Here, the phase difference image and the relationship between the object and the focus position F will be described with reference to FIGS. 5 to 11.

FIG. 5 is a diagram for describing an example of a camera 200A. FIG. 6 is a diagram illustrating an example of a captured image M captured by the camera 200A.

As illustrated in FIG. 5, the camera 200A includes a plurality of light receiving elements 210A and a plurality of lenses 220A respectively corresponding to the plurality of light receiving elements 210A. The light receiving element 210A receives light through the lens 220A and generates a pixel signal corresponding to the amount of received light.

The camera 200A generates, for example, the captured image M illustrated in FIG. 6 on the basis of the pixel signal. The captured image M includes, for example, an object Ob. As described above, a general monocular camera 200A has one light receiving element 210A per pixel. A pixel having one light receiving element as described above is also referred to as a normal pixel. The camera 200A generates one captured image M at a time.

Note that the camera 200A may have a color filter (illustration omitted) different for each pixel. For example, the camera 200A has a color filter of red (R), green (G), and blue (B), thereby generating a color captured image M.

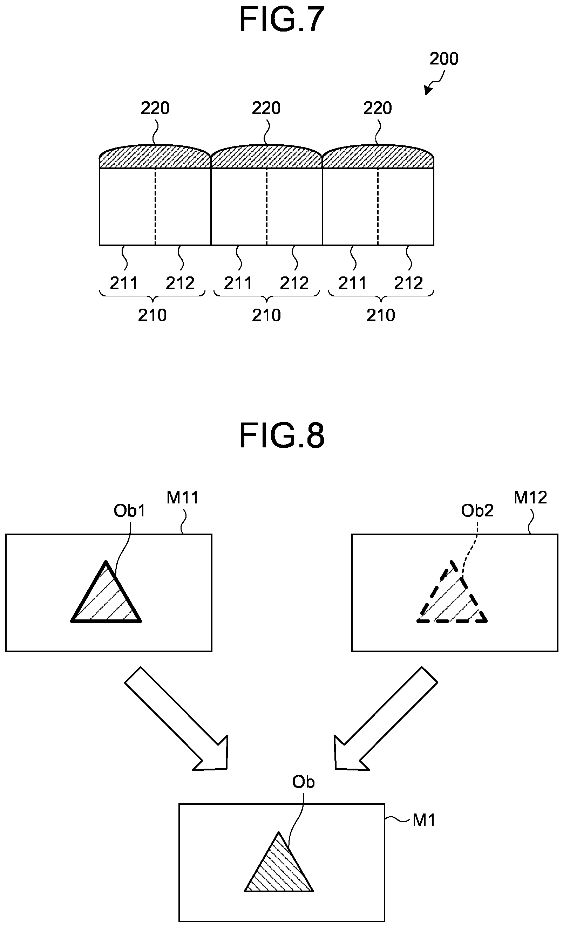

FIG. 7 is a diagram for describing an example of the image plane phase difference sensor 200 according to the first embodiment of the present disclosure. FIG. 8 is a diagram illustrating an example of a phase difference image captured by the image plane phase difference sensor 200.

As illustrated in FIG. 7, the image plane phase difference sensor 200 includes a plurality of pixels 210 and a plurality of lenses 220 respectively corresponding to the plurality of pixels 210. The pixel 210 includes a plurality of light receiving elements 211 and 212. A pixel having a plurality of light receiving elements as described above is also referred to as a phase difference pixel. The light receiving elements 211 and 212 receive light through the lens 220 and each generate a pixel signal corresponding to the amount of received light.

The image plane phase difference sensor 200 generates, for example, a phase difference image M11 (an example of a second phase difference image) illustrated in FIG. 8 on the basis of the pixel signal generated by the light receiving element 211. The phase difference image M11 includes, for example, an object Ob1.

The image plane phase difference sensor 200 generates, for example, a phase difference image M12 (an example of a 1 phase difference image) illustrated in FIG. 8 on the basis of the pixel signal generated by the light receiving element 212. The phase difference image M12 includes, for example, an object Ob2. Note that the object Ob2 included in the phase difference image M12 is the same object as the object Ob1 included in the phase difference image M11.

A captured image M1 illustrated in FIG. 8 is generated by combining the phase difference images M11 and M12. The captured image M1 may be generated by the image plane phase difference sensor 200 or may be generated by the information processing system.

Hereinafter, when the phase difference images M11 and M12 are distinguished, the phase difference image M11 may be referred to as a left image M11, and the phase difference image M12 may be referred to as a right image M12. When the phase difference images M11 and M12 are not distinguished, they are simply referred to as phase difference images.

As described above, the image plane phase difference sensor 200 includes the plurality of (two in FIG. 7) light receiving elements 211 and 212 per pixel. The image plane phase difference sensor 200 generates a plurality of phase difference images at a time.

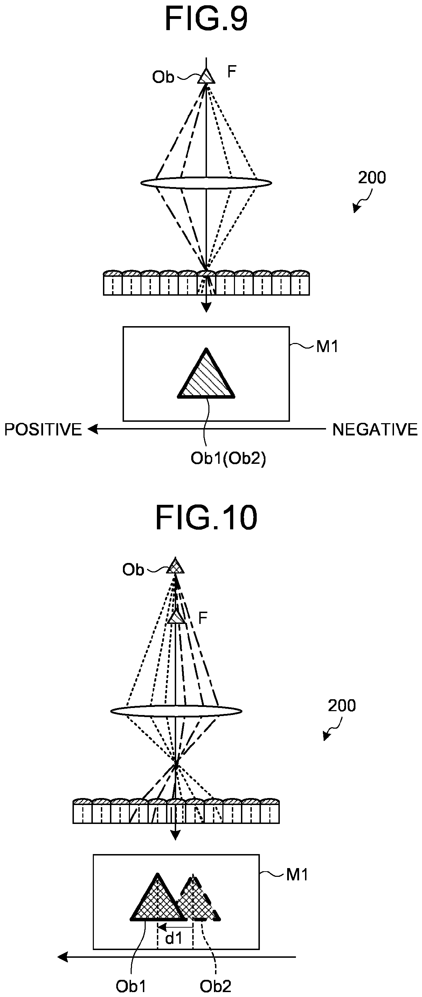

The position of the object Ob appearing in the phase difference image changes in the left and right images depending on the relationship between the object Ob and the focus position of the image plane phase difference sensor 200. Depending on the position of the object Ob with respect to the focus position, the deviation direction (parallax) of the position of the object Ob appearing in the left image M11 with respect to the position of the object Ob appearing in the right image M12 changes. Such a point will be described with reference to FIGS. 9 to 11.

FIG. 9 is a diagram for describing left and right images M11 and M12 in a case where an object is at a focus position.

As illustrated in FIG. 9, it is assumed that the object Ob is at the focus position F. That is, it is assumed that the image plane phase difference sensor 200 is focused on the object Ob. In this case, there is no deviation between the position of the object Ob1 in the left image M11 and the position of the object Ob2 in the right image M12. That is, the positions of the objects Ob1 and Ob2 become the same in the left image M11 and the right image M12, and no parallax occurs in the left and right images M11 and M12.

Note that, in the following, in a case where parallax (deviation direction) is described, a left direction in the drawing, that is, a direction in which the light receiving element 211 (see FIG. 7) is located in the pixel is a positive direction. In addition, a right direction in the drawing, that is, a direction in which the light receiving element 212 (see FIG. 7) is located in the pixel is a negative direction.

FIG. 10 is a diagram for describing left and right images M11 and M12 in a case where an object is farther with respect to a focus position.

As illustrated in FIG. 10, it is assumed that the object Ob is located farther with respect to the focus position F. In this case, the position of the object Ob1 in the left image M11 is deviated by a distance d1 in the positive direction from the position of the object Ob2 in the right image M12. That is, in the left image M11, parallax occurs in the positive direction with respect to the right image M12.

A state in which the object Ob is farther with respect to the focus position F is also referred to as "front focus". In the case of the front focus, the left image M11 is moved in the positive direction with respect to the right image M12. That is, the parallax between the left and right images M11 and M12 moves in the positive direction.

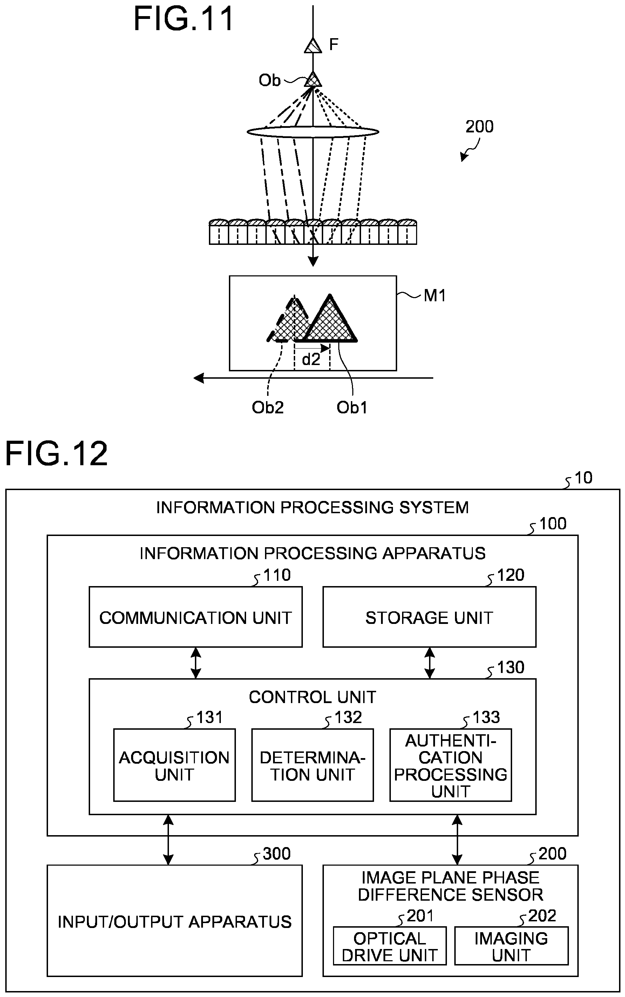

FIG. 11 is a diagram for describing left and right images M11 and M12 in a case where an object is closer with respect to a focus position.

As illustrated in FIG. 11, it is assumed that the object Ob is located closer with respect to the focus position F. In this case, the position of the object Ob1 in the left image M11 is deviated by a distance d2 in the negative direction from the position of the object Ob2 in the right image M12. That is, in the left image M11, parallax occurs in the negative direction with respect to the right image M12.

A state in which the object Ob is closer with respect to the focus position F is also referred to as "rear focus". In the case of the rear focus, the left image M11 is moved in the negative direction with respect to the right image M12. That is, the parallax between the left and right images M11 and M12 moves in the negative direction.

The information processing system according to the present embodiment detects the parallax direction of the left and right images M11 and M12 to determine whether the object Ob (for example, background Ob_B) is closer or farther with respect to the focus position F (front focus or rear focus).

The distance DF between the image plane phase difference sensor 200 and the focus position F is defined as a focus distance DF. In a case where the left image M11 is moved in the positive direction with respect to the right image M12, the information processing system determines that the object Ob is farther with respect to the focus distance DF and is the front focus. In a case where the left image M11 is moved in the negative direction with respect to the right image M12, the information processing system determines that the object Ob is closer with respect to the focus distance DF and is the rear focus.

As described above, the palm Ob_A is photographed at a position (rear focus) closer with respect to the focus distance DF. Therefore, in a case where the information processing system determines that even a part of the background region of the phase difference image is the rear focus, the information processing system determines that the impersonation by a third party has occurred. As described above, the information processing system can determine impersonation by detecting the parallax between the left and right images M11 and M12.

As described above, the information processing system according to the first embodiment of the present disclosure determines impersonation by detecting the parallax (deviation) between the left and right images M11 and M12. Since the light receiving elements 211 and 212 have a small left-right difference, it is difficult to calculate the depth with high accuracy from the parallax amount (distances d1 and d2). On the other hand, it is possible to accurately detect the parallax as to whether or not there is a parallax between the left and right images M11 and M12. The information processing system detects a parallax direction and determines impersonation. Therefore, the information processing system can determine impersonation with higher accuracy.

In addition, the depth measurement needs to be calculated for each pixel, which increases the calculation amount. On the other hand, the parallax between the left and right images M11 and M12 is detected not for each pixel but for each predetermined region as described below. Therefore, the information processing system can reduce the calculation amount for impersonation determination, and can determine impersonation at higher speed.

In the above-described example, the camera 200A and the image plane phase difference sensor 200 are different imaging apparatuses, but the camera 200A and the image plane phase difference sensor 200 may be achieved as one imaging apparatus. In this case, the imaging apparatus has a light receiving region in which both the normal pixel and the phase difference pixel are arranged. The imaging apparatus generates the captured image M on the basis of the pixel signal generated by the normal pixel. The imaging apparatus generates the phase difference image on the basis of the pixel signal generated by the phase difference pixel. The phase difference image is generally used to achieve an autofocus function of the imaging apparatus.

Since the information processing system determines impersonation using the image plane phase difference sensor 200, a sensor used for impersonation determination can be downsized, and an increase in size of the information processing system can be suppressed. In addition, the information processing system can determine impersonation without adding a new sensor, for example, by determining impersonation using the image plane phase difference sensor 200 mounted to achieve the autofocus function.

<1.2. Configuration Example of Information Processing System>

FIG. 12 is a block diagram illustrating a configuration example of an information processing system 10 according to the first embodiment of the present disclosure. The information processing system 10 is a system that performs non-contact biometric authentication. The information processing system 10 is a terminal apparatus used by a user, such as a smartphone or a tablet terminal. Alternatively, the information processing system 10 may be a mobile system such as a vehicle or a drone. Alternatively, the information processing system 10 may be an entrance/exit management system that is mounted at the entrance of a house or the like and manages entrance to and exit from a building or a room.

The information processing system 10 illustrated in FIG. 12 includes an information processing apparatus 100, the image plane phase difference sensor 200, and an input/output apparatus 300.

[Information Processing Apparatus 100]

The information processing apparatus 100 illustrated in FIG. 12 includes a communication unit 110, a storage unit 120, and a control unit 130.

(Communication Unit 110)

The communication unit 110 is a communication interface that communicates with an external apparatus via a network in a wired or wireless manner. The communication unit 110 is achieved by, for example, a network interface card (NIC) or the like. The communication unit 110 functions as a communication means of the information processing apparatus 100.

(Storage Unit 120)

The storage unit 120 is a storage apparatus capable of reading and writing data, such as DRAM, SRAM, flash memory, or a hard disk. The storage unit 120 functions as a storage means of the information processing apparatus 100.

(Control Unit 130)

The control unit 130 controls each unit of the information processing apparatus 100. The control unit 130 is achieved by, for example, a central processing unit (CPU), a micro processing unit (MPU), a graphics processing unit (GPU), or the like executing a program stored inside the information processing apparatus 100 using random access memory (RAM) or the like as a work area. In addition, the control unit 130 is achieved by, for example, an integrated circuit such as an application specific integrated circuit (ASIC) or a field programmable gate array (FPGA).

The control unit 130 includes an acquisition unit 131, a determination unit 132, and an authentication processing unit 133. Each block (acquisition unit 131 to authentication processing unit 133) constituting the control unit 130 is a functional block indicating a function of the control unit 130. These functional blocks may be software blocks or hardware blocks. For example, each of the functional blocks described above may be one software module achieved by software (including microprogram), or may be one circuit block on a semiconductor chip (die). Of course, each functional block may be one processor or one integrated circuit. The control unit 130 may be configured by a functional unit different from the above-described functional block. A configuration method of the functional block is arbitrary.

Note that the control unit 130 may be configured by a functional unit different from the above-described functional block. In addition, some or all of the operations of the blocks (the acquisition unit 131 to the authentication processing unit 133) constituting the control unit 130 may be performed by another apparatus. For example, some or all of the operations of the blocks constituting the control unit 130 may be performed by a control apparatus achieved by cloud computing.

(Acquisition Unit 131)

The acquisition unit 131 executes image acquisition processing (see FIG. 4). The acquisition unit 131 acquires the phase difference image from the image plane phase difference sensor 200.

(Determination Unit 132)

The determination unit 132 in FIG. 12 executes the impersonation determination processing (see FIG. 4). The determination unit 132 determines whether or not a person who executes the biometric authentication is impersonating on the basis of the phase difference image.

(Authentication Processing Unit 133)

The authentication processing unit 133 in FIG. 12 executes the authentication processing (see FIG. 4). In a case where the determination unit 132 determines that impersonation has not occurred, the authentication processing unit 133 executes authentication processing and determines whether or not a person who is to operate the information processing system 10 is a person who has authority to execute the operation.

[Image Plane Phase Difference Sensor 200]

The image plane phase difference sensor 200 is a sensor that generates a phase difference image around the information processing system 10. The image plane phase difference sensor 200 includes, for example, an optical drive unit 201 (an example of a drive unit) and an imaging unit 202.

The optical drive unit 201 includes a lens (illustration omitted) and a mechanism (illustration omitted) for driving the lens. The optical drive unit 201 performs so-called focus adjustment for adjusting the focus position F of the image plane phase difference sensor 200.

The imaging unit 202 images the surroundings of the information processing system 10. The imaging unit 202 includes, for example, the pixel 210 and the lens 220 in FIG. 7. The imaging unit 202 generates the left image M11 (an example of a second phase difference image) and the right image M12 (an example of a first phase difference image). The left image M11 and the right image M12 may be an RGB color image or a grayscale image.

In addition, the imaging unit 202 can generate a phase difference image by capturing a plurality of frames and averaging the frames. As a result, the image plane phase difference sensor 200 can reduce noise included in the phase difference image. Alternatively, the imaging unit 202 may output one of the plurality of frames as the phase difference image. For example, the imaging unit 202 can output an image having the maximum contrast among images of the plurality of frames as a phase difference image.

Note that, here, the imaging unit 202, that is, the image plane phase difference sensor 200 generates the phase difference image from the plurality of frames, but the phase difference image may be generated by those other than the image plane phase difference sensor 200. For example, the image plane phase difference sensor 200 may output a plurality of phase difference images, and the information processing apparatus 100 may generate a phase difference image used for the impersonation determination processing by averaging the plurality of phase difference images.

Note that, here, although the information processing system 10 includes the image plane phase difference sensor 200, the information processing system 10 may include a sensor other than the image plane phase difference sensor 200. For example, the information processing system 10 may include a sensor such as the camera 200A. The image plane phase difference sensor 200 may be used for focus adjustment of the camera 200A. For example, the optical drive unit 201 may function as the optical drive unit of the camera 200A.

[Input/Output Apparatus 300]

The input/output apparatus 300 is a user interface for exchanging information with the user. For example, the input/output apparatus 300 is an operation apparatus for the user to perform various operations, such as a keyboard, a mouse, an operation key, and a touch panel. The input/output apparatus 300 is a display apparatus such as a liquid crystal display or an organic electroluminescence (EL) display. The input/output apparatus 300 may be an acoustic apparatus such as a speaker or a buzzer. The input/output apparatus 300 may be a lighting apparatus such as a light emitting diode (LED) lamp. The input/output apparatus 300 functions as an input/output means (input means, output means, operation means, or notification means) of the information processing system 10.

The input/output apparatus 300 can present a determination result of the impersonation determination processing executed by the information processing apparatus 100 to the user on the basis of an instruction from the information processing apparatus 100. In addition, in a case where the image plane phase difference sensor 200 captures a palm image of the user, the input/output apparatus 300 can present the captured image M and guidance information for guiding the user to the user according to an instruction from the information processing apparatus 100.

<1.3. Information Processing>

Next, information processing executed by the information processing system 10 according to the first embodiment of the present disclosure will be described. As described above, the information processing system 10 executes the biometric authentication processing as the information processing.

There are a case where the information processing system 10 includes a display as the input/output apparatus 300 like a smartphone or a tablet terminal, and a case where the information processing system 10 does not include a display as the input/output apparatus 300 like a vehicle or an entrance of a house. First, the biometric authentication processing in a case where the information processing system 10 includes a display will be described.

(Biometric Authentication Processing)

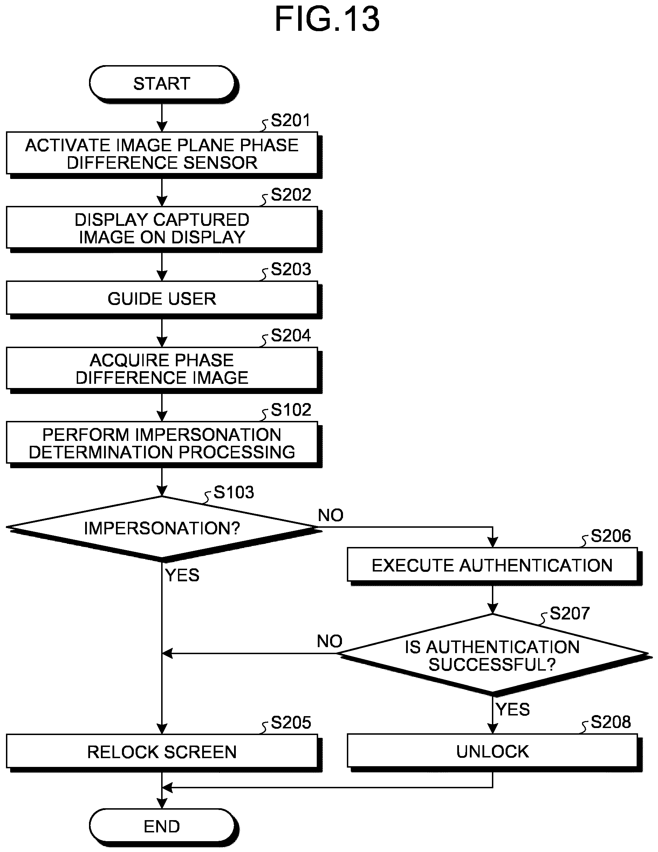

FIG. 13 is a flowchart illustrating a flow of an example of the biometric authentication processing according to the first embodiment of the present disclosure. The biometric authentication processing illustrated in FIG. 13 is executed by the information processing system 10 including a display like, for example, a smartphone. For example, the biometric authentication processing is executed when the user performs an operation to start the biometric authentication processing on the information processing system 10, such as holding a hand over the camera of the smartphone or pressing a button of the information processing system 10. Note that the same processing as the biometric authentication processing illustrated in FIG. 4 is denoted by the same reference sign, and description thereof is omitted.

As illustrated in FIG. 13, the information processing system 10 first activates the image plane phase difference sensor 200 (Step S201). The information processing system 10 displays the captured image M on the display (Step S202). The information processing system 10 may display the captured image M captured by the camera 200A on the display, or may display the captured image M1 obtained by combining the phase difference images on the display.

The information processing system 10 guides the user so that the background Ob_B and the palm Ob A are included in the phase difference image (Step S203). The information processing system 10 acquires the phase difference image from the image plane phase difference sensor 200 (Step S204). The processing of Steps S201 to S204 corresponds to, for example, the image acquisition processing of FIG. 4.

When it is determined that impersonation has occurred in the impersonation determination processing (Step S103; Yes), the information processing system 10 relocks the screen (Step S205), and ends the biometric authentication processing.

On the other hand, when it is determined that impersonation has not occurred in the impersonation determination processing (Step S103; No), the information processing system 10 executes authentication of the palm Ob_A (Step S206). For example, the information processing system 10 determines whether or not the palm Ob A of the captured image M matches the palm image stored in advance. Note that it is sufficient if the authentication is authentication using an image of the palm Ob_A, and various methods can be adopted.

The information processing system 10 determines whether or not the authentication is successful (Step S207). When the authentication has failed (Step S207; No), the information processing system 10 proceeds to Step S205. On the other hand, when the authentication is successful (Step S207; Yes), the information processing system 10 unlocks the screen (Step S208), and receives an operation from the user. The processing of Steps S205 to S208 corresponds to, for example, the authentication processing of FIG. 4.

Next, the biometric authentication processing in a case where the information processing system 10 does not include a display will be described.

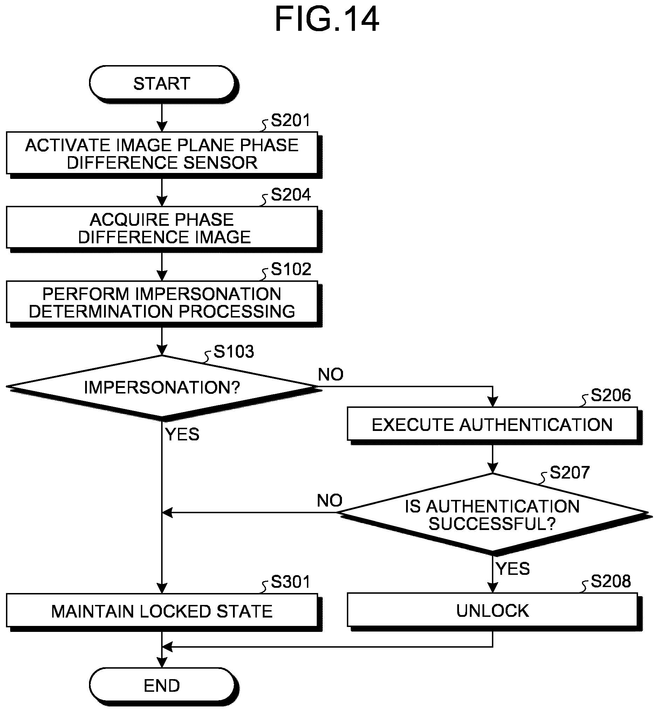

FIG. 14 is a flowchart illustrating a flow of another example of the biometric authentication processing according to the first embodiment of the present disclosure. The biometric authentication processing illustrated in FIG. 14 is executed by the information processing system 10 not including a display like, for example, a vehicle. For example, the biometric authentication processing is executed when the user performs an operation to start the biometric authentication processing on the information processing system 10, such as placing a hand on a door knob or pressing a button of the information processing system 10.

The biometric authentication processing illustrated in FIG. 14 is different from the biometric authentication processing illustrated in FIG. 13 in that the processing of Steps S202 and S203 is not executed. In addition, in a case where it is determined that impersonation has occurred in Step S103, the information processing system 10 maintains the locked state (Step S301). The other processing is the same as the biometric authentication processing illustrated in FIG. 13. Note that, in a case where it is determined in Step S103 that impersonation has occurred, the information processing system 10 may relock the screen as in Step S205 in FIG. 13.

Note that, although the processing of guiding the user by the information processing system 10 (processing in Step S203) is omitted here, the information processing system 10 may not omit the processing. For example, the information processing system 10 may guide the user by voice or the like.

(Guidance Processing)

As described above, the information processing system 10 according to the first embodiment of the present disclosure guides the user in order to acquire a desired phase difference image. Here, the desired phase difference image is a phase difference image in which both the palm Ob_A and the background Ob_B appear. For example, the information processing system 10 executes the guidance processing in order to guide the user.

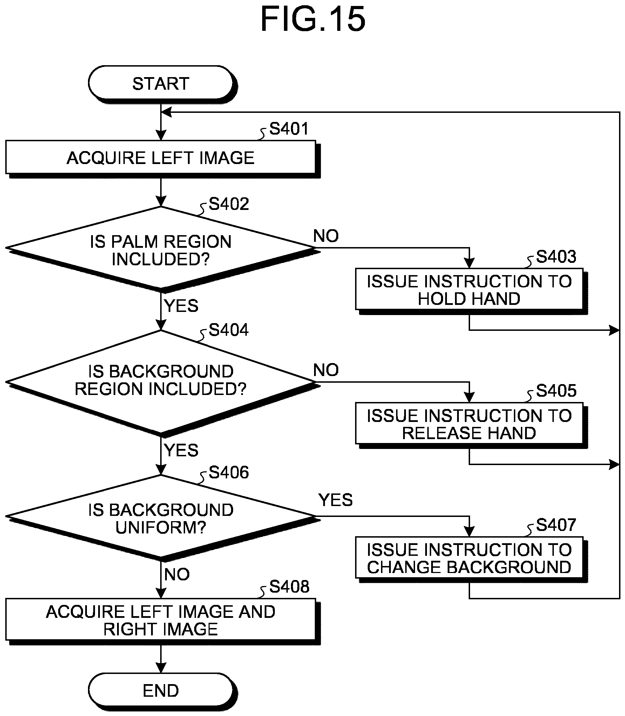

FIG. 15 is a flowchart illustrating a flow of an example of guidance processing according to the first embodiment of the present disclosure. The guidance processing is executed by the information processing apparatus 100 of the information processing system 10.

As illustrated in FIG. 15, the information processing apparatus 100 acquires the left image M11 from the image plane phase difference sensor 200 (Step S401). The information processing apparatus 100 determines whether or not a palm region is included in the left image M11 (Step S402).

In a case where the left image M11 does not include the palm region (Step S402; No), the information processing apparatus 100 issues an instruction to hold the hand of the user over the image plane phase difference sensor 200, for example, by presenting guidance information to the user (Step S403).

In a case where the left image M11 includes the palm region (Step S402; Yes), the information processing apparatus 100 determines whether or not a background region having a predetermined area or more is included in the left image M11 (Step S404).

In a case where the left image M11 does not include the background region having a predetermined area or more (Step S404; No), the information processing apparatus 100 issues an instruction to release the hand of the user from the image plane phase difference sensor 200, for example, by presenting guidance information to the user (Step S405).

In a case where the left image M11 includes the background region having a predetermined area or more (Step S404; Yes), the information processing apparatus 100 determines whether or not the background is uniform (Step S406).

When the background is uniform (Step S406; Yes), the information processing apparatus 100 issues an instruction to change the background, for example, by presenting guidance information to the user (Step S407).

When the background is not uniform (Step S406; No), the information processing apparatus 100 acquires the left image M11 and the right image M12 (Step S408), and ends the processing.

As described above, the information processing apparatus 100 executes the impersonation determination processing by detecting the deviation of the background regions between the left image M11 and the right image M12. For example, in a case where the background is uniform such as the background is a white wall, it becomes difficult for the information processing apparatus 100 to detect the deviation of the background region from the left image M11 and the right image M12. Therefore, in the present embodiment, the information processing apparatus 100 can execute the impersonation determination with higher accuracy by guiding the user so that the background is not uniform.

Note that, here, the information processing apparatus 100 guides the user on the basis of the left image M11, but the guidance method is not limited thereto. The information processing apparatus 100 may guide the user on the basis of the right image M12.

(Guidance Information)

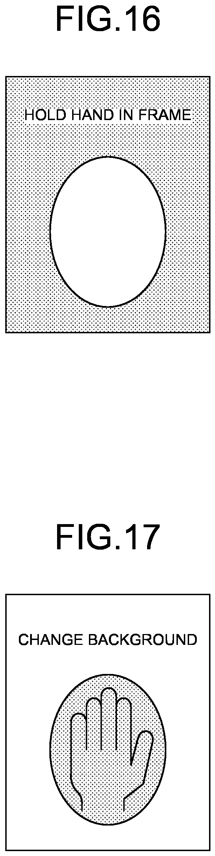

Next, an example of the guidance information will be described with reference to FIGS. 16 to 18.

FIG. 16 is a diagram illustrating an example of guidance information according to the first embodiment of the present disclosure. For example, in a case where the left image M11 does not include the palm region, the information processing apparatus 100 displays the guidance information of FIG. 16 on the display. Alternatively, for example, in a case where the palm is close to the image plane phase difference sensor 200 and the left image M11 does not include a background region having a predetermined area or more, the information processing apparatus 100 displays the guidance information of FIG. 16 on the display.

In the example of FIG. 16, the information processing apparatus 100 displays, on the display, guide information for guiding the position of the hand and character information for guiding the hand of the user to the guide information as the guidance information. Specifically, the information processing apparatus 100 displays a frame line indicating the position of the hand on the display as the guide information. In addition, the information processing apparatus 100 presents, on the display, character information issuing an instruction to hold a hand over the frame line.

Alternatively, in a case where the palm is close to the image plane phase difference sensor 200 and the left image M11 does not include a background region having a predetermined area or more, the information processing apparatus 100 may present, on the display, character information issuing an instruction to release the hand from the information processing system 10.

In addition, the information processing apparatus 100 may present the guidance information such that the palm becomes the rear focus, that is, the position of the hand of the user becomes closer to the image plane phase difference sensor 200 with respect to the focus position F. Specifically, the information processing apparatus 100 may adjust the size of the frame line or instruct the user to bring the hand close so that the palm becomes the rear focus.

As a result, the information processing apparatus 100 can guide the user so that both the palm and the background are captured.

FIG. 17 is a diagram illustrating another example of the guidance information according to the first embodiment of the present disclosure. For example, in a case where the background is uniform, the information processing apparatus 100 displays the guidance information of FIG. 17 on the display. In the example of FIG. 17, the information processing apparatus 100 displays character information issuing an instruction to change the background on the display. Alternatively, the information processing apparatus 100 may display character information instructing the user on the display such that an object or a pattern appears in the background.

As a result, the information processing apparatus 100 can acquire a phase difference image that facilitates background parallax detection, and can perform impersonation determination with higher accuracy.

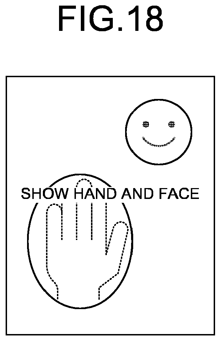

FIG. 18 is a diagram illustrating another example of the guidance information according to the first embodiment of the present disclosure. For example, in a case where the background is uniform, the information processing apparatus 100 displays the guidance information of FIG. 18 on the display.

In the example of FIG. 18, the information processing apparatus 100 issues an instruction not to change the background but to include a part of the living body (the face in the example of FIG. 18) other than the palm in the background. For example, the information processing apparatus 100 displays guide information for guiding the position and size of the face of the user on the display in addition to the guide information for guiding the position and size of the hand. The information processing apparatus 100 displays, on the display, character information for guiding the user so that the hand and the face of the user appear on the display.

In this manner, the information processing apparatus 100 guides the user so that an object other than the living body that is an authentication target is included in the background. As a result, the information processing apparatus 100 can acquire a phase difference image that facilitates background parallax detection, and can perform impersonation determination with higher accuracy.

Note that, in FIG. 18, the information processing apparatus 100 guides the user so that the background includes a living body other than the authentication target, but the guidance method by the information processing apparatus 100 is not limited thereto. For example, the information processing apparatus 100 may guide the user so as to appear an object other than a living body, such as a pillar, a patterned wall paper, or a personal item.

In addition, the information processing apparatus 100 may guide the user using voice information instead of the character information or in addition to the character information described above. By guiding the user using the voice information, the information processing apparatus 100 can more reliably guide the user even in a case where the user cannot confirm the display.

(Impersonation Determination Processing)

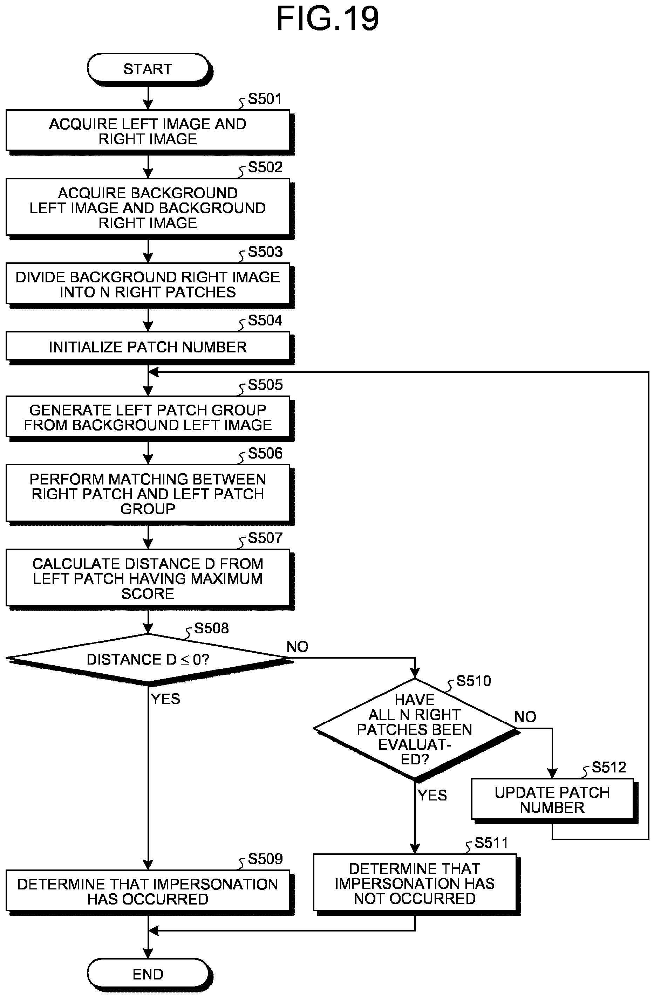

FIG. 19 is a flowchart illustrating an example of impersonation determination processing according to the first embodiment of the present disclosure. The impersonation determination processing illustrated in FIG. 19 is executed by the information processing apparatus 100. Note that, here, it is assumed that the palm is photographed at the rear focus. The information processing apparatus 100 divides the background region of the phase difference image into a plurality of patches, and determines that impersonation has occurred in a case where at least one of all the divided patches is the rear focus.

As illustrated in FIG. 19, the information processing apparatus 100 acquires the left image M11 and the right image M12 from the image plane phase difference sensor 200 (Step S501) .

The information processing apparatus 100 acquires the background regions of the left image M11 and the right image M12 as a background left image and a background right image (Step S502). For example, the information processing apparatus 100 first detects the palm region from the left image M11. The information processing apparatus 100 can detect the palm region from the left image M11 using, for example, skin color detection, deep neural network (DNN), or the like. Note that the method of detecting the palm region is not limited thereto. The information processing apparatus 100 can detect the palm region using an existing method. Similarly, the information processing apparatus 100 detects the palm region from the right image M12.

The information processing apparatus 100 sets the image in which the palm region of the left image M11 is masked as the background left image. The information processing apparatus 100 sets the image in which the palm region of the right image M12 is masked as the background right image.

Next, the information processing apparatus 100 divides the background right image into a grid shape, and generates N patches (hereinafter, also referred to as right patches. An example of a first region) (Step S503). Here, the size of the background right image is H * W (height H, width W). The information processing apparatus 100 divides the background right image into right patches $R_1, R_2, ..., R_N$ having a size of h * w (height h, width w). At this time, the center coordinates of the right patches $R_1, R_2, ... , R_N$ are expressed by Formula (1) described below. Note that N is N = p * q.

$$\{x_1, x_2 .... x_N\} = \left\{ \left(\frac{h}{2}, \frac{w}{2}\right), \left(\frac{h}{2} + h, \frac{w}{2}\right), ...., \left(\frac{h}{2} + h \times (p-1), \frac{w}{2} + w \times (q-1)\right) \right\}$$

$$\cdots (1)$$

The information processing apparatus 100 initializes the patch number (Step S504), and selects one from the N right patches R.

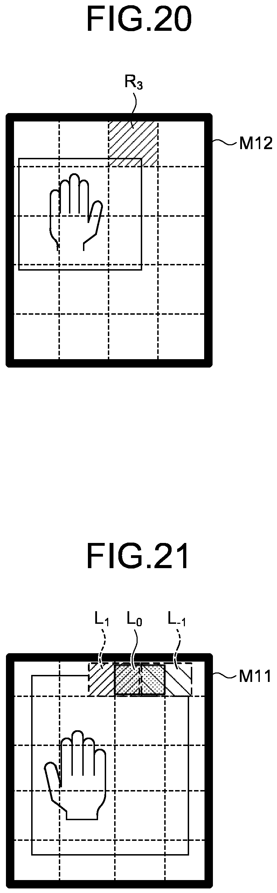

Here, FIG. 20 is a diagram for describing an example of right patches R according to the first embodiment of the present disclosure. Although FIG. 20 illustrates an example of the right patches R of the right image M12 instead of the background right image, the information processing apparatus 100 generates the right patches R in the background right image in which the palm region of the right image M12 is masked.

In FIG. 20, the information processing apparatus 100 generates $5 * 4 = 20$ right patches $R_1, R_2, ..., R_{20}$. That is, in the example of FIG. 20, N = 20, p = 5, and q = 4.

Here, it is assumed that the information processing apparatus 100 selects the right patch $R_3$ as the right patch R for calculating the parallax.

Referring back to FIG. 19, the information processing apparatus 100 generates a left patch group $\{L_{-n}, L_{-(n-1)}, ..., L_n\}$ including $2 * n$ patches (hereinafter, also referred to as left patches) from the background left image (Step S505). Note that the center coordinates of the left patch group $\{L_{-n}, L_{-(n-1)}, ..., L_n\}$ are $\{[x_i-n, y_i], [x_i-(n-1), y_i], ..., [x_i+n, y_i]\}$. Here, i is a patch number. $x_i$ is an x coordinate of the center of the right patch $R_3$. $y_i$ is a y coordinate of the center of the right patch $R_3$.

Here, FIG. 21 is a diagram for describing an example of a left patch group according to the first embodiment of the present disclosure. Although FIG. 21 illustrates an example of the left patch group of the left image M11 instead of the background left image, the information processing apparatus 100 generates the left patch group in the background left image in which the palm region of the left image M11 is masked.

As will be described below, the information processing apparatus 100 detects a deviation (parallax) between the right patch $R_1$ and the background left image in a horizontal direction (width direction) of the background left image. For example, the information processing apparatus 100 executes template matching of the background left image using the right patch $R_3$ as a template in the horizontal direction of the background left image. The information processing apparatus 100 generates a left patch group as a target for template matching. Note that the horizontal direction is an array direction of the light receiving elements 211 and 212 (see FIG. 7) included in one pixel of the image plane phase difference sensor 200.

In the example of FIG. 21, the information processing apparatus 100 generates a left patch group including left patches $L_1, L_0,$ and $L_{-1}$ arranged in the same row as the right patch $R_3$ in the background left image. Note that the size of the background left image is $H * W$ (height H, width W), which is the same as the size of the background right image. The size of the left patch L is $h * w$ (height h, width w), which is the same as the size of the right patch R. As illustrated in FIG. 21, a part of the left patch $L_1$ overlaps with a part of the left patch Lo. In addition, a part of the left patch Lo overlaps with a part of the left patch $L_{-1}$. Note that, in FIG. 21, the left patch $L_1$ is illustrated as a rectangular region hatched with left-downward oblique lines in a dotted frame. The left patch Lo is illustrated as a rectangular region hatched with dots in a solid frame. The left patch $L_{-1}$ is illustrated as a rectangular region hatched with right-downward oblique lines in a one-dot chain line frame. In this manner, the left patch L can be arranged so as to overlap at least a part of another left patch L.

Referring back to FIG. 19, the information processing apparatus 100 uses the right patch $R_3$ as a template, and executes template matching of the left patch group $\{L_{-1}, L_0, L_1\}$ generated in Step S505 (Step S506). For example, the information processing apparatus 100 executes template matching with the right patch $R_3$ for each of the left patches $L_{-1}, L_0,$ and $L_1$ included in the left patch group $\{L_{-1}, L_0, L_1\}$, and calculates scores $S_{-1}, S_0,$ and $S_1$. Examples of a method of calculating a score used for template matching include sum of squared difference (SSD), normalized cross correlation (NCC), and phase-only correlation (POC), but are not limited thereto.

The information processing apparatus 100 calculates a distance D between the left patch L (an example of a second region) having the maximum calculated score S and the right patch R (Step S507). In the examples of FIGS. 20 and 21, it is assumed that the score $S_{-1}$ of the left patch $L_{-1}$ is the maximum. In this case, the information processing apparatus 100 calculates D = -1 as the distance D between the left patch $L_{-1}$ and the right patch $R_3$.

In this manner, the information processing apparatus 100 sets the moving direction (positive direction or negative direction) in which the score S is the maximum as the horizontal movement direction of the right patch R. In a case where the scores S are the same in all the left patches L of the left patch group, the information processing apparatus 100 sets the maximum score to $S_0$ and the movement amount (distance D) to zero.

The information processing apparatus 100 determines whether or not the distance D is zero or less (Step S508). In a case where the distance D is zero or less (Step S508; Yes), the information processing apparatus 100 determines that impersonation has occurred (Step S509).

When the distance D is less than zero, that is, a negative value, the corresponding right patch R and left patch L are in the rear focus state. When the distance D is zero, the corresponding right patch R and left patch L are focused, and there is no parallax between the right patch R and the left patch L.

In a case where at least a part of the background (right patch R, left patch L) is closer to the image plane phase difference sensor 200 with respect to the focus position F (the distance D is smaller than zero) or is the same as the focus position F (the distance D is zero), the information processing apparatus 100 determines that impersonation has occurred.

In a case where the distance D is larger than zero (Step S508; No), the information processing apparatus 100 determines whether or not all the N right patches R have been evaluated (Step S510). That is, the information processing apparatus 100 executes template matching with the left patch group for all the right patches R, and determines whether or not impersonation determination has been performed. Note that when the distance D is larger than zero, that is, a positive value, the corresponding right patch R and left patch L are in the front focus state.

When all the N right patches R have been evaluated (Step S510; Yes), the information processing apparatus 100 determines that impersonation has not occurred (Step S511). On the other hand, in a case where there is a right patch R that has not been evaluated yet (Step S510; No), the information processing apparatus 100 updates the patch number to be evaluated (Step S512), and evaluates the right patch R that has not been evaluated.

Note that, here, the information processing apparatus 100 determines that impersonation has occurred in a case where the distance D is equal to or less than zero, but a condition for determining impersonation is not limited thereto. For example, the information processing apparatus 100 may determine that impersonation has occurred in a case where the distance D is smaller than zero. That is, in a case where the palm is photographed in the rear focus state, the information processing apparatus 100 may determine that impersonation has occurred in a case where at least a part of the background is the rear focus.

As described above, the information processing apparatus 100 according to the present embodiment performs authentication of a living body (for example, palm) in a non-contact manner by using an image. The information processing apparatus 100 includes the control unit 130 (see FIG. 12). The acquisition unit 131 of the control unit 130 acquires the phase difference image (for example, left image M11 and right image M12) acquired by the image plane phase difference sensor 200.

The determination unit 132 of the control unit 130 detects a background (for example, background left image and background right image) from the phase difference image. The determination unit 132 determines that impersonation has occurred when determining that at least a part of the background is the same as the focus position F of the image plane phase difference sensor 200 or is closer to the image plane phase difference sensor 200 with respect to the focus position F.

As a result, the information processing apparatus 100 does not need to detect the depth of the phase difference image, and can perform the impersonation determination with higher accuracy and at higher speed.

<<2. Second Embodiment>>

In the first embodiment described above, the information processing apparatus 100 performs evaluation by template matching on all of the divided N right patches R, but it is not limited thereto. For example, the information processing apparatus 100 may perform evaluation by template matching on M (M < N) right patches R among the N right patches R.

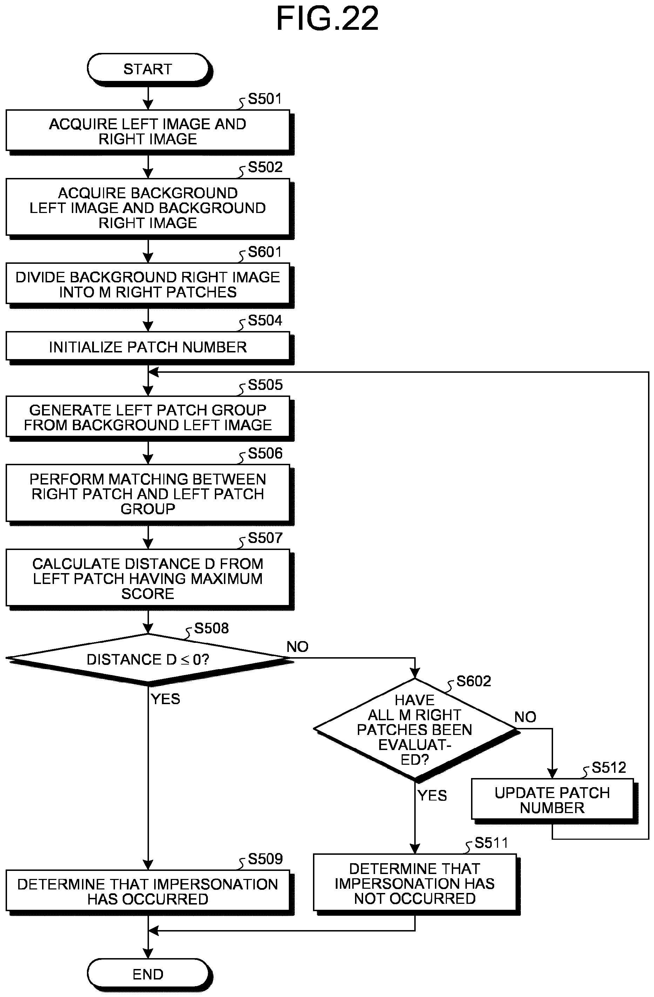

FIG. 22 is a flowchart illustrating an example of impersonation determination processing according to the second embodiment of the present disclosure. The impersonation determination processing illustrated in FIG. 22 is executed by the information processing apparatus 100. Note that, here, it is assumed that the palm is photographed at the rear focus. Note that the same processing as the impersonation determination processing illustrated in FIG. 19 is denoted by the same reference sign, and description thereof is omitted.

As illustrated in FIG. 22, the information processing apparatus 100 divides the background right image into N right patches R, and selects M right patches R from the divided N right patches R (Step S601). For example, the information processing apparatus 100 arbitrarily selects M right patches R.

The information processing apparatus 100 executes template matching with the left patch group for the arbitrarily selected M right patches R to calculate the distance D. The information processing apparatus 100 determines impersonation according to the value of the distance D.

The information processing apparatus 100 determines whether or not all the M right patches R have been evaluated (Step S602). When all the M right patches R have been evaluated (Step S602; Yes), the information processing apparatus 100 determines that impersonation has not occurred (Step S511). On the other hand, in a case where there is a right patch R that has not been evaluated yet (Step S602; No), the information processing apparatus 100 updates the patch number to be evaluated (Step S512), and evaluates the right patch R that has not been evaluated.

As described above, the information processing apparatus 100 according to the present embodiment executes evaluation by template matching on an arbitrary patch (for example, right patch R). As a result, the information processing apparatus 100 can reduce the calculation amount of the impersonation determination processing while maintaining the accuracy of the impersonation determination processing. The information processing apparatus 100 can execute the impersonation determination processing at higher speed.

<<3. Third Embodiment>>

In the first embodiment described above, the information processing apparatus 100 evaluates the right patch R on the basis of the distance D between the left patch L having the maximum score S and the right patch R, but the reference used by the information processing apparatus 100 for the evaluation is not limited to the distance D. For example, the information processing apparatus 100 may evaluate the right patch R using the total value of the scores S.

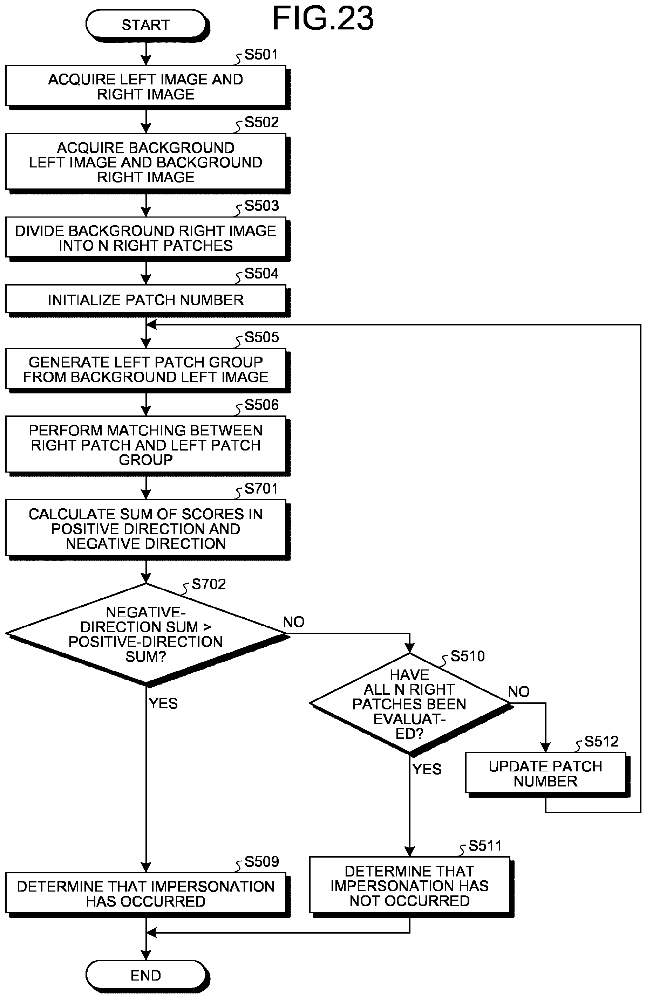

FIG. 23 is a flowchart illustrating an example of impersonation determination processing according to the third embodiment of the present disclosure. The impersonation determination processing illustrated in FIG. 23 is executed by the information processing apparatus 100. Note that, here, it is assumed that the palm is photographed at the rear focus. Note that the same processing as the impersonation determination processing illustrated in FIG. 19 is denoted by the same reference sign, and description thereof is omitted.

In Step S506, the information processing apparatus 100 that has executed template matching between the right patch R and each left patch L of the left patch group and calculated the scores S calculates the sum of the scores S in the positive direction and the negative direction (Step S701).

The information processing apparatus 100 calculates the total value (hereinafter, also referred to as a positive-direction sum) of the scores $S_1, S_2, \ldots, S_n$ of the left patches $L_1, L_2, \ldots, L_n$ located in the positive direction with respect to the right patch $R_i$.

The information processing apparatus 100 calculates the total value (hereinafter, also referred to as a negative-direction sum) of the scores $S_{-n}, S_{-(n-1)}, \ldots, S_{-1}$ of the left patches $L_{-n}, L_{-(n-1)}, \ldots, L_{-1}$ located in the negative direction with respect to the right patch $R_i$.

The information processing apparatus 100 sets a direction in which the sum is larger between the positive-direction sum and the negative-direction sum as the horizontal movement direction of the right patch $R_i$. Note that, for example, in a case where the background is a flat surface without texture, the positive-direction sum and the negative-direction sum coincide with each other.

Next, the information processing apparatus 100 determines whether or not the negative-direction sum is larger than the positive-direction sum (Step S702). In a case where the negative-direction sum is larger than the positive-direction sum (Step S702; Yes), the information processing apparatus 100 determines that impersonation has occurred (Step S509).

When the negative-direction sum is larger than the positive-direction sum, the horizontal movement direction of the right patch is the positive direction, and the corresponding right patch R and left patch L are in the rear focus state.

On the other hand, in a case where the positive-direction sum is equal to or larger than the negative-direction sum (Step S702; No), the information processing apparatus 100 determines whether or not all the N right patches R have been evaluated (Step S510). When the positive-direction sum is equal to or larger than the negative-direction sum, the horizontal movement direction of the right patch is the negative direction, and the corresponding right patch R and left patch L are in the front focus state.

As described above, the information processing apparatus 100 according to the present embodiment determines impersonation according to the total value of the scores S in the positive or negative direction. In this manner, the information processing apparatus 100 can perform the impersonation determination using the total value of the scores S instead of the distance D. The information processing apparatus 100 adds the results of the template matching with the plurality of left patches L and detects the deviation (parallax) direction between the background right image and the background right image, so that the information processing apparatus 100 can more stably determine impersonation.

<<4. Fourth Embodiment>>

In each of the above-described embodiments, a feature (for example, an edge or the like. Hereinafter, also referred to as background information) for detecting the parallax is included in the background region of the phase difference image, and the information processing apparatus 100 determines impersonation using the background information. However, the background information included in the background region of the phase difference image may be insufficient.

For example, in a case where a non-impersonating user performs biometric authentication, such as a case where the user performs biometric authentication in a place where background information is insufficient, such as wallpaper with less texture, background information included in the phase difference image may be insufficient. In this way, for example, in a case where the background information included in the phase difference image is insufficient, such as no edge exists in the background region, the information processing apparatus 100 can determine that the user performing the biometric authentication is not impersonating.

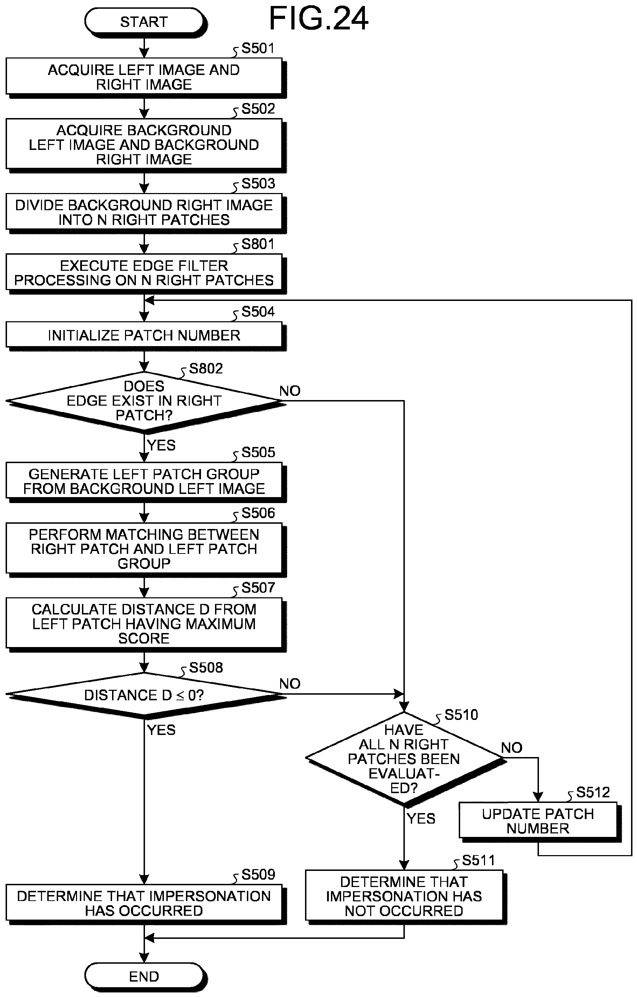

FIG. 24 is a flowchart illustrating an example of impersonation determination processing according to the fourth embodiment of the present disclosure. The impersonation determination processing illustrated in FIG. 24 is executed by the information processing apparatus 100. Note that, here, it is assumed that the palm is photographed at the rear focus. Note that the same processing as the impersonation determination processing illustrated in FIG. 19 is denoted by the same reference sign, and description thereof is omitted.

When the background right image is divided into N right patches R in Step S503, the information processing apparatus 100 executes edge filter processing on the N right patches R (Step S801). In this manner, the information processing apparatus 100 executes processing of emphasizing the edge of the right patch R.

The information processing apparatus 100 initializes the patch number (Step S504), and selects one from the N right patches R.

The information processing apparatus 100 determines whether or not an edge exists in the selected right patch R (Step S802). When no edge exists in the right patch R (Step S802; No), the information processing apparatus 100 proceeds to Step S510.

On the other hand, when an edge exists in the right patch R (Step S802; Yes), the information processing apparatus 100 proceeds to Step S505 and executes template matching between the right patch R and the left patch group.

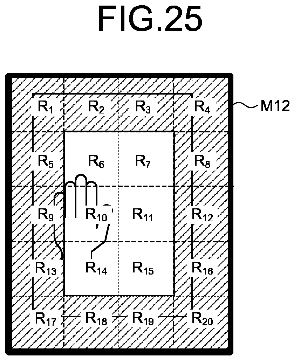

FIG. 25 is a diagram illustrating an example of right patches R for which the information processing apparatus 100 according to the fourth embodiment of the present disclosure executes pattern matching. Although FIG. 25 illustrates an example of the right patches R of the right image M12 instead of the background right image, the information processing apparatus 100 generates the right patches R in the background right image in which the palm region of the right image M12 is masked.

As described above, the information processing apparatus 100 executes template matching on the right patches R in which an edge other than the palm exists. In the example of FIG. 25, the information processing apparatus 100 executes template matching on right patches $R_1$ to $R_5$, $R_8$, $R_9$, $R_{12}$, $R_{13}$, and $R_{16}$ to $R_{20}$ in which edges other than the palm exist among right patches $R_1$ to $R_{20}$.

In this manner, the information processing apparatus 100 executes template matching on the right patches R in which an edge exists. As a result, the information processing apparatus 100 can perform the impersonation determination (evaluation) only in the region considered to be effective for the impersonation determination within the phase difference image, and can further reduce the calculation amount of the impersonation determination processing.

In addition, in a case where no edge exists in all the right patches R, the information processing apparatus 100 determines that impersonation has not occurred. As a result, the information processing apparatus 100 can execute the impersonation determination processing even in a case where the background information included in the phase difference image is insufficient.

<<5. Fifth Embodiment>>

In the above-described fourth embodiment, the information processing apparatus 100 determines that impersonation has not occurred when the background information included in the background region of the phase difference image is insufficient.

On the other hand, for example, in a case where a third party impersonates a person in question, such as a case where the third party prints a photograph of the living body on a large paper that does not fit in the angle of view of the camera, or a case where the living body is displayed on a large display, the background information included in the phase difference image may be insufficient. In this way, for example, in a case where the background information included in the phase difference image is insufficient, such as no edge exists in the background region, the information processing apparatus 100 can determine that the third party performing the biometric authentication is impersonating.

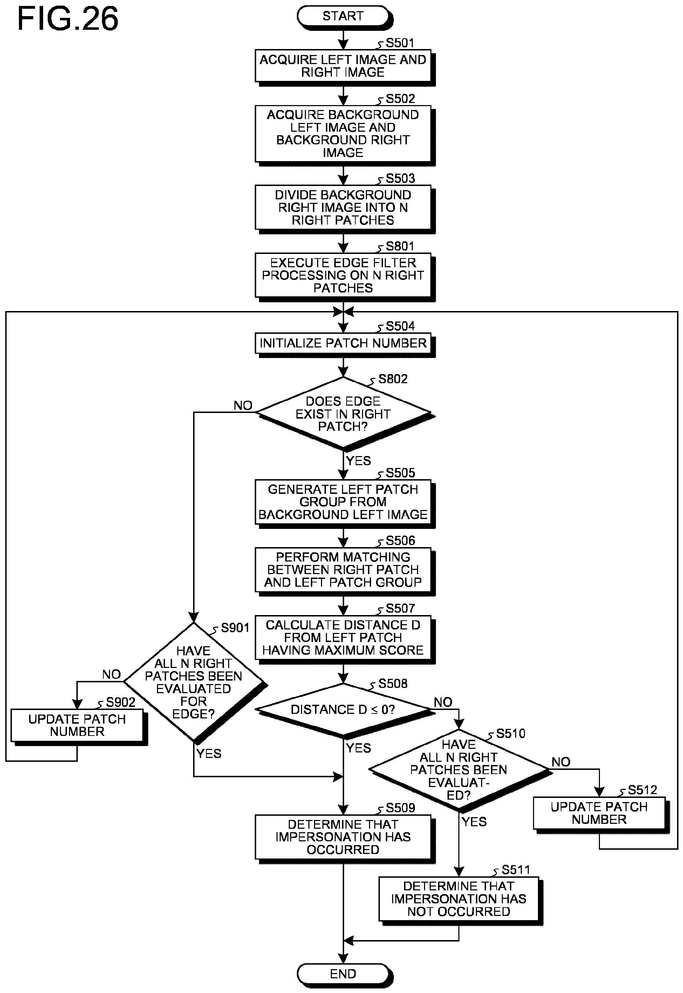

FIG. 26 is a flowchart illustrating an example of impersonation determination processing according to the fifth embodiment of the present disclosure. The impersonation determination processing illustrated in FIG. 26 is executed by the information processing apparatus 100. Note that, here, it is assumed that the palm is photographed at the rear focus. Note that the same processing as the impersonation determination processing illustrated in FIG. 24 is denoted by the same reference sign, and description thereof is omitted.

The information processing apparatus 100 determines whether or not an edge exists in the selected right patch R (Step S802). When an edge exists in the right patch R (Step S802; Yes), the information processing apparatus 100 proceeds to Step S505 and executes template matching between the right patch R and the left patch group.

On the other hand, in a case where no edge exists in the right patch R (Step S802; No), the information processing apparatus 100 determines whether or not all the N right patches R have been evaluated for edge (Step S901). That is, the information processing apparatus 100 determines whether or not it has been determined whether or not an edge exists in all the N right patches R.

When all the N right patches R have been evaluated for edge (Step S901; Yes), the information processing apparatus 100 determines that impersonation has occurred (Step S509). On the other hand, in a case where there is a right patch R that has not been evaluated yet for the existence of an edge (Step S901; No), the information processing apparatus 100 updates the patch number to be evaluated (Step S902), and evaluates the right patch R that has not been evaluated.

In this manner, the information processing apparatus 100 executes template matching on the right patches R in which an edge exists. As a result, the information processing apparatus 100 can perform the impersonation determination (evaluation) only in the region considered to be effective for the impersonation determination within the phase difference image, and can further reduce the calculation amount of the impersonation determination processing.

In addition, in a case where no edge exists in all the right patches R, the information processing apparatus 100 determines that impersonation has occurred. As a result, the information processing apparatus 100 can execute the impersonation determination processing even in a case where the background information included in the phase difference image is insufficient.

<<6. Sixth Embodiment>>

In each of the above-described embodiments, the information processing apparatus 100 executes the impersonation determination processing by detecting a deviation between the left image and the right image captured by the image plane phase difference sensor 200. In the present embodiment, the information processing apparatus 100 executes impersonation determination by detecting a material or an item used for impersonation (hereinafter, also referred to as impersonation material), such as paper, the display of a smartphone or the like, an impersonation mask, or the like in addition to the detection of the deviation.

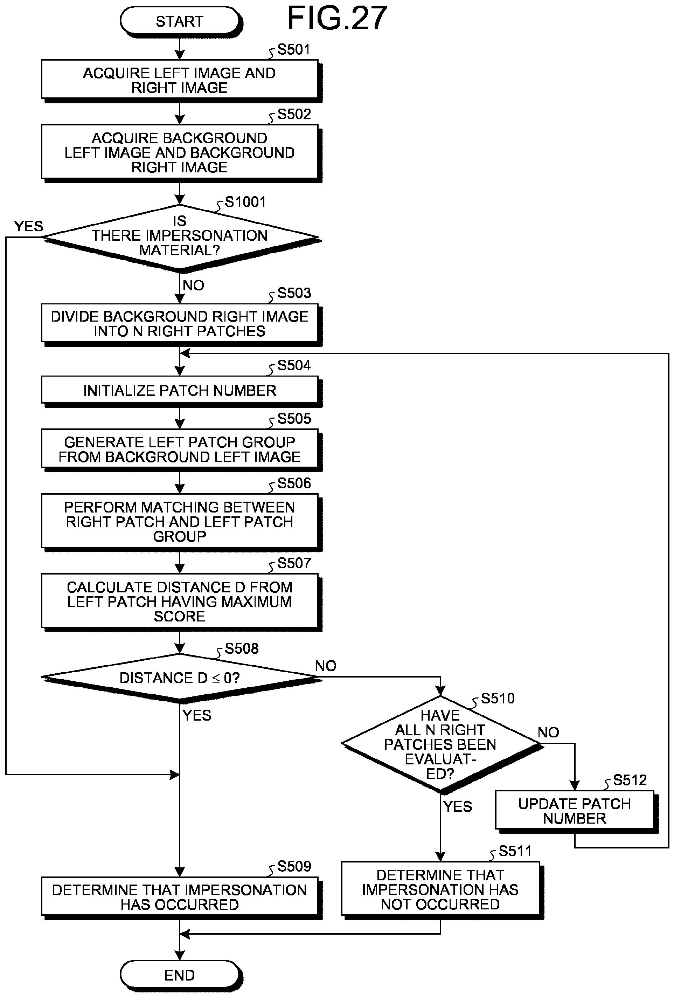

FIG. 27 is a flowchart illustrating an example of impersonation determination processing according to the sixth embodiment of the present disclosure. The impersonation determination processing illustrated in FIG. 27 is executed by the information processing apparatus 100. Note that, here, it is assumed that the palm is photographed at the rear focus. Note that the same processing as the impersonation determination processing illustrated in FIG. 19 is denoted by the same reference sign, and description thereof is omitted.

The information processing apparatus 100 that has acquired the background left image and the background right image in Step S502 determines whether or not there is an impersonation material on the basis of at least one of the background left image and the background right image (Step S1001). For example, the information processing apparatus 100 determines whether or not an impersonation material is included in the background right image.

The information processing apparatus 100 can determine whether or not an impersonation material appears in the background right image by using a method such as object detection or object recognition such as DNN, for example.

When there is no impersonation material (Step S1001; No), the information processing apparatus 100 proceeds to Step S503 and determines impersonation using the phase difference image. On the other hand, when there is an impersonation material (Step S1001; Yes), the information processing apparatus 100 proceeds to Step S509 and determines impersonation.

As described above, the information processing apparatus 100 detects an impersonation material before performing impersonation evaluation by template matching. When an impersonation material is detected, the information processing apparatus 100 determines that impersonation has occurred without performing evaluation by template matching. As a result, the information processing apparatus 100 can further reduce the calculation amount of the impersonation determination processing.

<<7. Seventh Embodiment>>

In each of the above-described embodiments, the phase difference image is captured at a position where the palm is closer to the image plane phase difference sensor 200 with respect to the focus position F, but the position of the palm may be the same as the focus position F. For example, as described above, it is assumed that the optical drive unit 201 (see FIG. 12) of the image plane phase difference sensor 200 can adjust the focus position F of the image plane phase difference sensor 200, that is, can adjust the focus of the image plane phase difference sensor 200. In this case, the image plane phase difference sensor 200 can acquire the phase difference image by focusing on the palm (by bringing the palm into focus).

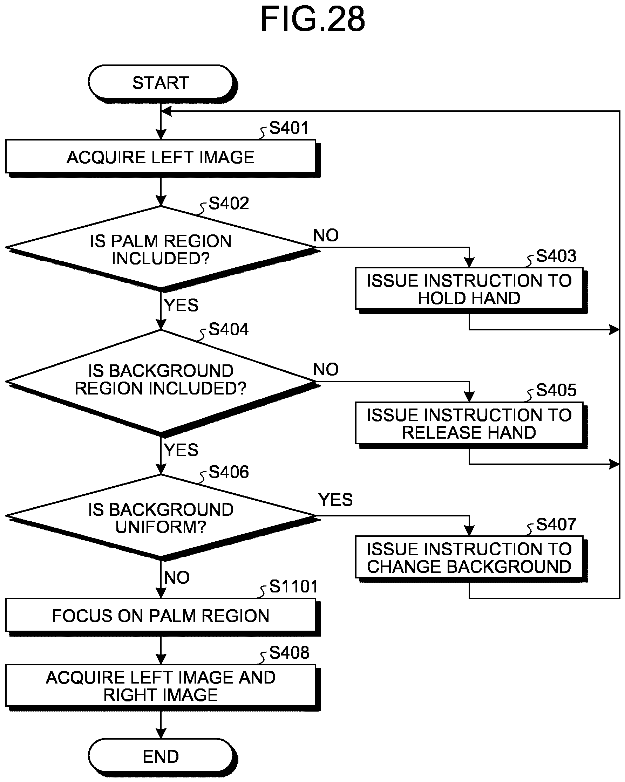

FIG. 28 is a flowchart illustrating an example of a flow of guidance processing according to the seventh embodiment of the present disclosure. The guidance processing of FIG. 28 is executed by the information processing apparatus 100. Note that the same processing as the guidance processing illustrated in FIG. 15 is denoted by the same reference sign, and description thereof is omitted.

The information processing apparatus 100 that has determined that the background is not uniform (Step S406; Yes) instructs the image plane phase difference sensor 200 to focus on the palm region (Step S1101). As a result, the image plane phase difference sensor 200 performs the focus adjustment so as to focus on the palm.

Next, the information processing apparatus 100 acquires the left image M11 and the right image M12 (Step S408). As a result, the information processing apparatus 100 can acquire the left image M11 and the right image M12 focused on the palm.

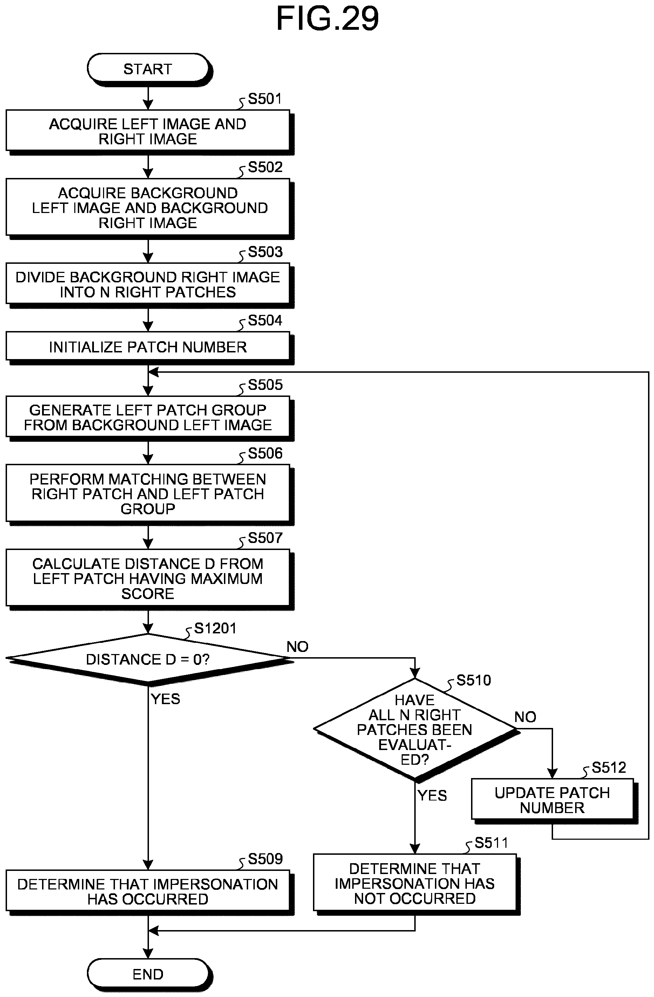

FIG. 29 is a flowchart illustrating an example of a flow of impersonation determination processing according to the seventh embodiment of the present disclosure. The impersonation determination processing illustrated in FIG. 29 is executed by the information processing apparatus 100. Note that the same processing as the impersonation determination processing illustrated in FIG. 19 is denoted by the same reference sign, and description thereof is omitted.

The information processing apparatus 100 that has calculated the distance D in Step S507 determines whether or not the distance D is zero (Step S1201). As described above, when the distance D is zero, the corresponding right patch R and left patch L are focused, and there is no parallax between the right patch R and the left patch L. That is, a part (right patch R, left patch L) of the background is the same as the focus position F.

Here, as described above, the information processing apparatus 100 acquires the phase difference image focused on the palm. That is, the fact that at least a part (right patch R, left patch L) of the background is the same as the focus position F means that at least a part of the background is on the same plane as the palm.

In this case, that is, in a case where the distance D is zero (Step S1201; Yes), the information processing apparatus 100 proceeds to Step S509 and determines that impersonation has occurred. On the other hand, when the distance D is not zero (Step S1201; No), the information processing apparatus 100 proceeds to Step S510.

As described above, the information processing system 10 adjusts the focus so as to focus on the palm and then acquires the phase difference image, so that the information processing apparatus 100 can execute the impersonation determination processing using the phase difference image focused on the palm. As a result, the information processing system 10 can execute the impersonation determination processing regardless of the positional relationship between the palm and the image plane phase difference sensor 200.

Note that, here, the information processing system 10 adjusts the focus so as to focus on the palm, but the focus adjustment by the information processing system 10 is not limited thereto. For example, the information processing system 10 can adjust the focus such that the palm becomes the rear focus.

<<8. Other Embodiments>>

The processing according to the embodiments described above may be performed in various different forms other than the embodiments described above.

In the above embodiments, the information processing apparatus 100 divides the background right image into the N right patches R, and detects the deviation from the background left image for each right patch R, but the information processing apparatus 100 may divide the background left image into N left patches L. In this case, the information processing apparatus 100 divides the background left image into N left patches L, and detects a deviation from the background right image for each of the left patches L. In this manner, the information processing apparatus 100 can execute the processing by switching the left and right phase difference images in the above embodiments.

Among the pieces of processing described in the aforementioned embodiments, all or some of the pieces of processing described as being performed automatically can be performed manually, or all or some of the pieces of processing described as being performed manually can be performed automatically by a known method. Additionally, the processing procedures, the specific names, and the information including various data and parameters indicated in the aforementioned document and drawings can be arbitrarily changed unless otherwise specified. For example, the various types of information illustrated in each drawing are not limited to the illustrated information.

In addition, each component of each apparatus illustrated in the drawings is functionally conceptual, and is not necessarily physically configured as illustrated in the drawings. That is, a specific form of distribution and integration of apparatuses is not limited to those illustrated, and all or a part thereof can be functionally or physically distributed and integrated in an arbitrary unit according to various loads, usage situations, and the like. Note that this configuration by distribution and integration may be performed dynamically.

In addition, the above-described embodiments can be appropriately combined within an area not contradicting processing contents. In addition, the order of each step illustrated in the flowchart and sequence diagram of the above-described embodiments can be changed as appropriate.

In addition, for example, the present embodiments can be implemented as any configuration constituting an apparatus or a system, for example, a processor as a system large scale integration (LSI) or the like, a module using a plurality of processors or the like, a unit using a plurality of modules or the like, a set obtained by further adding other functions to a unit, or the like (that is, a configuration of a part of the apparatus).

Note that, in the present embodiments, the system means a set of a plurality of components (apparatuses, modules (parts), and the like), and it does not matter whether or not all the components are in the same housing. Accordingly, a plurality of apparatuses housed in separate housings and connected via a network and one apparatus in which a plurality of modules is housed in one housing are both the system.

In addition, for example, the present embodiment can adopt a configuration of cloud computing in which one function is shared and processed by a plurality of apparatuses in cooperation via a network.

<<9. Hardware Configuration>>

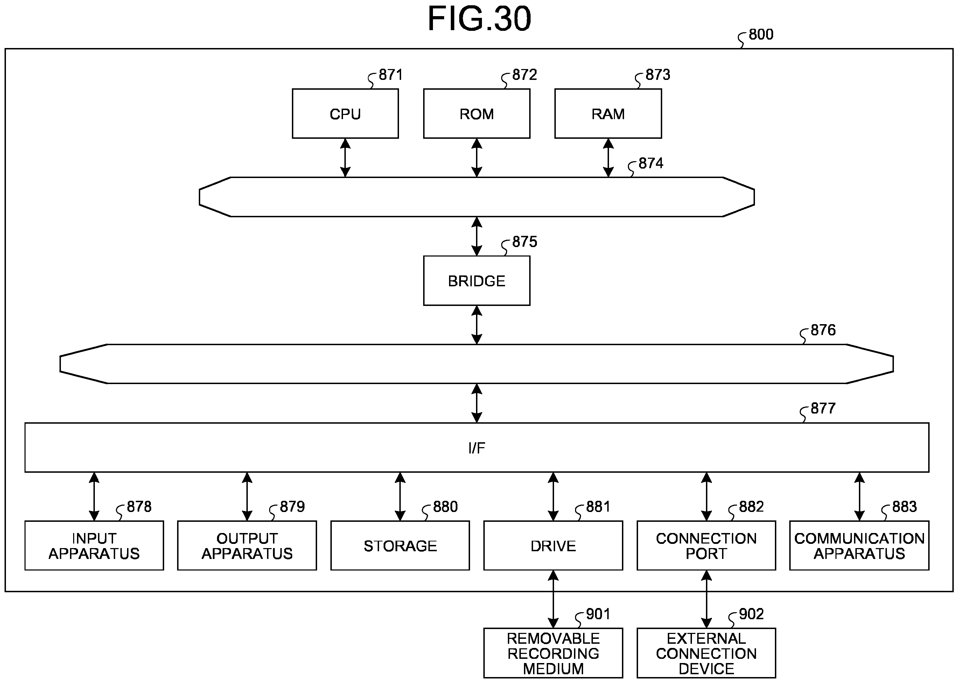

Finally, a hardware configuration of the information processing apparatus according to the present embodiment will be described with reference to FIG. 30. FIG. 30 is a block diagram illustrating an example of a hardware configuration of an information processing apparatus 800 according to the present embodiment. Note that the information processing apparatus 800 illustrated in FIG. 30 can achieve, for example, the information processing system 10. Information processing by the information processing system 10 according to the present embodiment is achieved by cooperation of software and hardware described below.

As illustrated in FIG. 30, the information processing apparatus 800 includes, for example, a CPU 871, ROM 872, RAM 873, a host bus 874, a bridge 875, an external bus 876, an interface 877, an input apparatus 878, an output apparatus 879, a storage 880, a drive 881, a connection port 882, and a communication apparatus 883. Note that the hardware configuration illustrated here is an example, and some of the components may be omitted. In addition, components other than the components illustrated here may be further included.

(CPU 871)

The CPU 871 functions as, for example, an operation processing apparatus or a control apparatus, and controls the entire operation or part of the operation of each component on the basis of various programs recorded in the ROM 872, the RAM 873, the storage 880, or a removable recording medium 901.

Specifically, the CPU 871 achieves operation processing in the information processing system 10.

(ROM 872, RAM 873)

The ROM 872 is a means that stores a program read by the CPU 871, data used for operation, and the like. The RAM 873 temporarily or permanently stores, for example, a program read by the CPU 871, various parameters that appropriately change when the program is executed, and the like.

(Host Bus 874, Bridge 875, External Bus 876, Interface 877)

The CPU 871, the ROM 872, and the RAM 873 are mutually connected via, for example, the host bus 874 capable of high-speed data transmission. On the other hand, the host bus 874 is connected to the external bus 876 having a relatively low data transmission speed via the bridge 875, for example. In addition, the external bus 876 is connected to various components via the interface 877.

(Input Apparatus 878)

As the input apparatus 878, for example, a mouse, a keyboard, a touch panel, a button, a switch, a lever, and the like are used. Further, as the input apparatus 878, a remote controller capable of transmitting a control signal using infrared rays or other radio waves (hereinafter, remote controller) may be used. In addition, the input apparatus 878 includes a voice input apparatus such as a microphone.

(Output Apparatus 879)

The output apparatus 879 is an apparatus capable of visually or audibly notifying the user of acquired information, such as a display apparatus such as a cathode ray tube (CRT), an LCD, or an organic EL, an audio output apparatus such as a speaker or a headphone, a printer, a mobile phone, or a facsimile. In addition, the output apparatus 879 according to the present disclosure includes various vibration devices capable of outputting tactile stimulation.

(Storage 880)

The storage 880 is an apparatus for storing various data. As the storage 880, for example, a magnetic storage device such as a hard disk drive (HDD), a semiconductor storage device, an optical storage device, a magneto-optical storage device, or the like is used.

(Drive 881)

The drive 881 is, for example, an apparatus that reads information recorded on the removable recording medium 901 such as a magnetic disk, an optical disk, a magneto-optical disk, or a semiconductor memory, or writes information to the removable recording medium 901.

(Removable Recording Medium 901)

The removable recording medium 901 is, for example, a DVD medium, a Blu-ray (registered trademark) medium, an HD DVD medium, various semiconductor storage media, or the like. Of course, the removable recording medium 901 may be, for example, an IC card on which a non-contact IC chip is mounted, an electronic device, or the like.

(Connection Port 882)

The connection port 882 is a port for connecting an external connection device 902 such as a universal serial bus (USB) port, an IEEE 1394 port, a small computer system interface (SCSI), an RS-232C port, or an optical audio terminal.

(External Connection Device 902)

The external connection device 902 is, for example, a printer, a portable music player, a digital camera, a digital video camera, an IC recorder, or the like.

(Communication Apparatus 883)

The communication apparatus 883 is a communication device for connecting to a network, and is, for example, a communication card for wired or wireless LAN, Wi-Fi (registered trademark), Bluetooth (registered trademark), or wireless USB (WUSB), a router for optical communication, a router for asymmetric digital subscriber line (ADSL), a modem for various communications, or the like.

<<10. Conclusion>>

While the preferred embodiments of the present disclosure have been described in detail above with reference to the accompanying drawings, the present technology is not limited to such examples. It will be apparent to those skilled in the art of the present disclosure that various modifications and alterations can be conceived within the scope of the technical idea described in the claims and naturally fall within the technical scope of the present disclosure.
For example, it is also possible to create a computer program for causing hardware such as the CPU, the ROM, and the RAM built in the information processing system 10 described above to exhibit the functions of the information processing system 10. In addition, a computer-readable storage medium storing the computer program is also provided.
In addition, the effects described in the present specification are merely illustrative or exemplary and are not limiting. That is, the technology according to the present disclosure can have other effects apparent to those skilled in the art from the description of the present specification, in addition to or instead of the above effects.
Note that the present technology can also have the following configurations.

(1) An information processing apparatus that performs non-contact authentication of a living body using an image, the information processing apparatus comprising:

a control unit that
acquires a phase difference image acquired by an image plane phase difference sensor,
detects a background from the phase difference image, and
determines whether or not impersonation has occurred on a basis of a comparison with a focus position of the image plane phase difference sensor in at least a part of the background.

(2) The information processing apparatus according to (1), wherein
the control unit determines that impersonation has occurred when determining that at least a part of the background is same as the focus position of the image plane phase difference sensor or is closer to the image plane phase difference sensor with respect to the focus position.
(3) The information processing apparatus according to (1) or (2), wherein

the control unit
acquires a first phase difference image and a second phase difference image as the phase difference image, and
determines whether or not at least a part of the background is same as the focus position or closer to the image plane phase difference sensor with respect to the focus position according to a deviation between the background in the first phase difference image and the background in the second phase difference image.

(4) The information processing apparatus according to (3), wherein

the control unit
divides the background of the first phase difference image into a plurality of first regions, and

determines whether or not the background of the first region is same as the focus position or is closer to the image plane phase difference sensor with respect to the focus position according to a distance between the first region and a second region of the second phase difference image corresponding to the first region.

(5) The information processing apparatus according to any one of (1) to (4), wherein the control unit determines that impersonation has not occurred in a case where background information of the phase difference image is insufficient.

(6) The information processing apparatus according to any one of (1) to (4), wherein the control unit determines that impersonation has occurred in a case where background information of the phase difference image is insufficient.

(7) The information processing apparatus according to any one of (1) to (6), wherein in a case where background information is included in the background of the phase difference image, the control unit determines whether or not a region including the background information is same as the focus position or is closer to the image plane phase difference sensor with respect to the focus position.

(8) The information processing apparatus according to any one of (1) to (7), wherein the control unit guides a user so that the phase difference image includes the living body and the background.

(9) The information processing apparatus according to any one of (1) to (8), wherein the control unit notifies a user to change the background.

(10) The information processing apparatus according to any one of (1) to (9), wherein the control unit guides the user so that a part of the user other than the living body used for the authentication is included in the background.

(11) The information processing apparatus according to any one of (1) to (10), wherein

the control unit
instructs a drive unit to drive a lens of the image plane phase difference sensor so as to focus on the living body, and
acquires the phase difference image focused on the living body from the image plane phase difference sensor.

(12) The information processing apparatus according to any one of (1) to (11), wherein the control unit does not perform the authentication of the living body when determining that impersonation has occurred.

(13) An information processing system that performs non-contact authentication of a living body using an image, the information processing system comprising:

an image plane phase difference sensor that generates a phase difference image of the living body; and
an information processing apparatus that determines whether or not the living body impersonates, wherein
the information processing apparatus includes
a control unit that
detects a background from the phase difference image, and
determines whether or not impersonation has occurred on a basis of a comparison with a focus position of the image plane phase difference sensor in at least a part of the background.

(14) An information processing method executed by a processor when performing non-contact authentication of a living body using an image, the information processing method comprising:

acquiring a phase difference image acquired by an image plane phase difference sensor;
detecting a background from the phase difference image; and
determining whether or not impersonation has occurred on a basis of a comparison with a focus position of the image plane phase difference sensor in at least a part of the background.

(15) A program causing a computer when performing non-contact authentication of a living body using an image to execute:

acquiring a phase difference image acquired by an image plane phase difference sensor;
detecting a background from the phase difference image; and
determining whether or not impersonation has occurred on a basis of a comparison with a focus position of the image plane phase difference sensor in at least a part of the background.

Reference Signs List

10      INFORMATION PROCESSING SYSTEM

100     INFORMATION PROCESSING APPARATUS

110     COMMUNICATION UNIT

120     STORAGE UNIT

130     CONTROL UNIT

131     ACQUISITION UNIT

132     DETERMINATION UNIT

133     AUTHENTICATION PROCESSING UNIT

200     IMAGE PLANE PHASE DIFFERENCE SENSOR

300     INPUT/OUTPUT APPARATUS

**Claims**

1.  An information processing apparatus that performs non-contact authentication of a living body using an image, the information processing apparatus comprising:

    a control unit that
    acquires a phase difference image acquired by an image plane phase difference sensor,
    detects a background from the phase difference image, and
    determines whether or not impersonation has occurred on a basis of a comparison with a focus position of the image plane phase difference sensor in at least a part of the background.

2.  The information processing apparatus according to claim 1, wherein
    the control unit determines that impersonation has occurred when determining that at least a part of the background is same as the focus position of the image plane phase difference sensor or is closer to the image plane phase difference sensor with respect to the focus position.

3.  The information processing apparatus according to claim 1, wherein

    the control unit
    acquires a first phase difference image and a second phase difference image as the phase difference image, and
    determines whether or not at least a part of the background is same as the focus position or closer to the image plane phase difference sensor with respect to the focus position according to a deviation between the background in the first phase difference image and the background in the second phase difference image.

4.  The information processing apparatus according to claim 3, wherein

    the control unit
    divides the background of the first phase difference image into a plurality of first regions, and
    determines whether or not the background of the first region is same as the focus position or is closer to the image plane phase difference sensor with respect to the focus position according to a distance between the first region and a second region of the second phase difference image corresponding to the first region.

5.  The information processing apparatus according to claim 1, wherein the control unit determines that impersonation has not occurred in a case where background information of the phase difference image is insufficient.

6.  The information processing apparatus according to claim 1, wherein the control unit determines that impersonation has occurred in a case where background information of the phase difference image is insufficient.

**7.** The information processing apparatus according to claim 1, wherein in a case where background information is included in the background of the phase difference image, the control unit determines whether or not a region including the background information is same as the focus position or is closer to the image plane phase difference sensor with respect to the focus position.

**8.** The information processing apparatus according to claim 1, wherein the control unit guides a user so that the phase difference image includes the living body and the background.

**9.** The information processing apparatus according to claim 1, wherein the control unit notifies a user to change the background.

**10.** The information processing apparatus according to claim 1, wherein the control unit guides the user so that a part of the user other than the living body used for the authentication is included in the background.

**11.** The information processing apparatus according to claim 1, wherein

the control unit
instructs a drive unit to drive a lens of the image plane phase difference sensor so as to focus on the living body, and
acquires the phase difference image focused on the living body from the image plane phase difference sensor.

**12.** The information processing apparatus according to claim 1, wherein the control unit does not perform the authentication of the living body when determining that impersonation has occurred.

**13.** An information processing system that performs non-contact authentication of a living body using an image, the information processing system comprising:

an image plane phase difference sensor that generates a phase difference image of the living body; and
an information processing apparatus that determines whether or not the living body impersonates, wherein
the information processing apparatus includes
a control unit that
detects a background from the phase difference image, and
determines whether or not impersonation has occurred on a basis of a comparison with a focus position of the image plane phase difference sensor in at least a part of the background.

**14.** An information processing method executed by a processor when performing non-contact authentication of a living body using an image, the information processing method comprising:

acquiring a phase difference image acquired by an image plane phase difference sensor;
detecting a background from the phase difference image; and
determining whether or not impersonation has occurred on a basis of a comparison with a focus position of the image plane phase difference sensor in at least a part of the background.

**15.** A program causing a computer when performing non-contact authentication of a living body using an image to execute:

acquiring a phase difference image acquired by an image plane phase difference sensor;
detecting a background from the phase difference image; and
determining whether or not impersonation has occurred on a basis of a comparison with a focus position of the image plane phase difference sensor in at least a part of the background.

# FIG.1

# FIG.2

# FIG.3

F

DF

DA=DB

200

# FIG.4

START

PERFORM IMAGE ACQUISITION
PROCESSING ⌐S101

PERFORM IMPERSONATION
DETERMINATION PROCESSING ⌐S102

IMPERSONATION? ⌐S103

NO

YES

PERFORM
AUTHENTICATION
PROCESSING ⌐S104

END

# FIG.5

200A

220A 220A 220A

210A 210A 210A

# FIG.6

Ob

M

# FIG.7

200

220    220    220

211  212   211  212   211  212

210      210      210

# FIG.8

M11    Ob1

Ob2    M12

Ob

M1

# FIG.9

Ob ~ F

200

POSITIVE ← ─────────────────── NEGATIVE

Ob1(Ob2)

M1

# FIG.10

Ob ~

F

200

M1

d1

Ob1          Ob2

# FIG.11

# FIG.12

INFORMATION PROCESSING SYSTEM ⌇10

INFORMATION PROCESSING APPARATUS ⌇100

| COMMUNICATION UNIT ⌇110 | STORAGE UNIT ⌇120 |
|---|---|

CONTROL UNIT ⌇130

| ACQUISITION UNIT ⌇131 | DETERMINA-TION UNIT ⌇132 | AUTHENTI-CATION PROCESSING UNIT ⌇133 |
|---|---|---|

| INPUT/OUTPUT APPARATUS ⌇300 | IMAGE PLANE PHASE DIFFERENCE SENSOR ⌇200 |
|---|---|
| | OPTICAL DRIVE UNIT ⌇201 / IMAGING UNIT ⌇202 |

# FIG.13

```
              ┌─────────────┐
              │    START    │
              └──────┬──────┘
                     │              ⌠S201
        ┌────────────▼────────────┐
        │ ACTIVATE IMAGE PLANE PHASE│
        │    DIFFERENCE SENSOR     │
        └────────────┬────────────┘
                     │              ⌠S202
        ┌────────────▼────────────┐
        │    DISPLAY CAPTURED      │
        │    IMAGE ON DISPLAY      │
        └────────────┬────────────┘
                     │              ⌠S203
        ┌────────────▼────────────┐
        │       GUIDE USER         │
        └────────────┬────────────┘
                     │              ⌠S204
        ┌────────────▼────────────┐
        │     ACQUIRE PHASE        │
        │    DIFFERENCE IMAGE      │
        └────────────┬────────────┘
                     │              ⌠S102
        ┌────────────▼────────────┐
        │ PERFORM IMPERSONATION    │
        │ DETERMINATION PROCESSING │
        └────────────┬────────────┘
                     │              ⌠S103
                    ◇──────────── NO
          IMPERSONATION?
                    ◇
                    │ YES
```

ACTIVATE IMAGE PLANE PHASE DIFFERENCE SENSOR — S201

DISPLAY CAPTURED IMAGE ON DISPLAY — S202

GUIDE USER — S203

ACQUIRE PHASE DIFFERENCE IMAGE — S204

PERFORM IMPERSONATION DETERMINATION PROCESSING — S102

IMPERSONATION? — S103

NO → EXECUTE AUTHENTICATION — S206

IS AUTHENTICATION SUCCESSFUL? — S207

NO

YES

RELOCK SCREEN — S205

UNLOCK — S208

END

# FIG.14

```
        ┌─────────────┐
        │    START     │
        └──────┬───────┘
               │            ⌐S201
    ┌──────────▼──────────────┐
    │ ACTIVATE IMAGE PLANE     │
    │ PHASE DIFFERENCE SENSOR  │
    └──────────┬──────────────┘
               │            ⌐S204
    ┌──────────▼──────────────┐
    │   ACQUIRE PHASE          │
    │   DIFFERENCE IMAGE       │
    └──────────┬──────────────┘
               │            ⌐S102
    ┌──────────▼──────────────┐
    │ PERFORM IMPERSONATION    │
    │ DETERMINATION PROCESSING │
    └──────────┬──────────────┘
               │     ⌐S103
          ◇─────────────◇  ──NO──┐
          │ IMPERSONATION? │      │        ⌐S206
          ◇──────┬───────◇       ▼
               YES│          ┌──────────────────────┐
                  │          │ EXECUTE AUTHENTICATION│
                  │          └──────────┬───────────┘
                  │                     │      ⌐S207
                  │              ◇───────────────◇
                  ◄────NO────────│ IS AUTHENTICATION│
                  │              │   SUCCESSFUL?    │
                  │              ◇───────┬─────────◇
                  │                    YES│   ⌐S208
        ⌐S301     │                 ┌─────▼─────┐
    ┌─────────────▼──┐              │  UNLOCK   │
    │ MAINTAIN LOCKED │             └─────┬─────┘
    │     STATE       │                   │
    └────────┬────────┘◄──────────────────┘
             │
        ┌────▼────┐
        │   END    │
        └─────────┘
```

# FIG.15

```
                    ┌─────────────┐
                    │    START    │
                    └─────────────┘
                          │
                          ▼          S401
                ┌──────────────────────┐
                │  ACQUIRE LEFT IMAGE   │
                └──────────────────────┘
                          │
                          ▼          S402
                     ◇─────────◇         NO
                    IS PALM REGION ──────────────┐
                     INCLUDED?                    │       S403
                     ◇─────────◇          ┌──────────────────────┐
                          │ YES           │ ISSUE INSTRUCTION TO  │
                          ▼          S404  │     HOLD HAND         │
                     ◇─────────◇          └──────────────────────┘
                    IS BACKGROUND    NO
                   REGION INCLUDED? ───────────┐
                     ◇─────────◇                │       S405
                          │ YES           ┌──────────────────────┐
                          ▼          S406  │ ISSUE INSTRUCTION TO  │
                     ◇─────────◇          │    RELEASE HAND       │
                    IS BACKGROUND    YES  └──────────────────────┘
                     UNIFORM? ──────────────┐
                     ◇─────────◇             │       S407
                          │ NO         ┌──────────────────────┐
                          ▼     S408    │ ISSUE INSTRUCTION TO  │
                ┌──────────────────────┐│  CHANGE BACKGROUND    │
                │ ACQUIRE LEFT IMAGE AND│└──────────────────────┘
                │     RIGHT IMAGE       │
                └──────────────────────┘
                          │
                          ▼
                    ┌─────────────┐
                    │     END     │
                    └─────────────┘
```

# FIG.16

HOLD HAND IN FRAME

# FIG.17

CHANGE BACKGROUND

# FIG.18

SHOW HAND AND FACE

# FIG.19

START

**S501**
ACQUIRE LEFT IMAGE AND RIGHT IMAGE

**S502**
ACQUIRE BACKGROUND LEFT IMAGE AND BACKGROUND RIGHT IMAGE

**S503**
DIVIDE BACKGROUND RIGHT IMAGE INTO N RIGHT PATCHES

**S504**
INITIALIZE PATCH NUMBER

**S505**
GENERATE LEFT PATCH GROUP FROM BACKGROUND LEFT IMAGE

**S506**
PERFORM MATCHING BETWEEN RIGHT PATCH AND LEFT PATCH GROUP

**S507**
CALCULATE DISTANCE D FROM LEFT PATCH HAVING MAXIMUM SCORE

**S508**
DISTANCE D ≤ 0?

NO →

**S510**
HAVE ALL N RIGHT PATCHES BEEN EVALUATED?

NO →

**S512**
UPDATE PATCH NUMBER

YES ↓ (from S508)

**S509**
DETERMINE THAT IMPERSONATION HAS OCCURRED

YES ↓ (from S510)

**S511**
DETERMINE THAT IMPERSONATION HAS NOT OCCURRED

END

# FIG.20

# FIG.21

# FIG.22

```
                    START

                      │ S501
        ┌─────────────▼─────────────┐
        │   ACQUIRE LEFT IMAGE AND   │
        │        RIGHT IMAGE         │
        └─────────────┬─────────────┘
                      │ S502
        ┌─────────────▼─────────────┐
        │   ACQUIRE BACKGROUND       │
        │ LEFT IMAGE AND BACKGROUND  │
        │        RIGHT IMAGE         │
        └─────────────┬─────────────┘
                      │ S601
        ┌─────────────▼─────────────┐
        │ DIVIDE BACKGROUND RIGHT    │
        │ IMAGE INTO M RIGHT PATCHES │
        └─────────────┬─────────────┘
                      │ S504
        ┌─────────────▼─────────────┐
        │   INITIALIZE PATCH NUMBER  │
        └─────────────┬─────────────┘
                      │ S505
        ┌─────────────▼─────────────┐
        │   GENERATE LEFT PATCH      │
        │ GROUP FROM BACKGROUND      │
        │       LEFT IMAGE           │
        └─────────────┬─────────────┘
                      │ S506
        ┌─────────────▼─────────────┐
        │ PERFORM MATCHING BETWEEN   │
        │ RIGHT PATCH AND LEFT PATCH │
        │           GROUP            │
        └─────────────┬─────────────┘
                      │ S507
        ┌─────────────▼─────────────┐
        │ CALCULATE DISTANCE D FROM  │
        │ LEFT PATCH HAVING MAXIMUM  │
        │           SCORE            │
        └─────────────┬─────────────┘
                      │ S508
```

DISTANCE D ≤ 0?  — NO →  S602 — HAVE ALL M RIGHT PATCHES BEEN EVALUATED? — NO → S512 UPDATE PATCH NUMBER

YES

S602 YES → S511 DETERMINE THAT IMPERSONATION HAS NOT OCCURRED

S509 DETERMINE THAT IMPERSONATION HAS OCCURRED

```
                    END
```

# FIG.23

START

↓ S501

ACQUIRE LEFT IMAGE AND RIGHT IMAGE

↓ S502

ACQUIRE BACKGROUND LEFT IMAGE AND BACKGROUND RIGHT IMAGE

↓ S503

DIVIDE BACKGROUND RIGHT IMAGE INTO N RIGHT PATCHES

↓ S504

INITIALIZE PATCH NUMBER

↓ S505

GENERATE LEFT PATCH GROUP FROM BACKGROUND LEFT IMAGE

↓ S506

PERFORM MATCHING BETWEEN RIGHT PATCH AND LEFT PATCH GROUP

↓ S701

CALCULATE SUM OF SCORES IN POSITIVE DIRECTION AND NEGATIVE DIRECTION

↓ S702

NEGATIVE-DIRECTION SUM > POSITIVE-DIRECTION SUM?

NO → S510

HAVE ALL N RIGHT PATCHES BEEN EVALUATED?

NO → S512

UPDATE PATCH NUMBER

YES ↓ (S702)

S509

DETERMINE THAT IMPERSONATION HAS OCCURRED

YES ↓ (S510)

S511

DETERMINE THAT IMPERSONATION HAS NOT OCCURRED

↓

END

# FIG.24

START

ACQUIRE LEFT IMAGE AND RIGHT IMAGE ⌇S501

ACQUIRE BACKGROUND LEFT IMAGE AND BACKGROUND RIGHT IMAGE ⌇S502

DIVIDE BACKGROUND RIGHT IMAGE INTO N RIGHT PATCHES ⌇S503

EXECUTE EDGE FILTER PROCESSING ON N RIGHT PATCHES ⌇S801

INITIALIZE PATCH NUMBER ⌇S504

DOES EDGE EXIST IN RIGHT PATCH? ⌇S802 — NO

YES

GENERATE LEFT PATCH GROUP FROM BACKGROUND LEFT IMAGE ⌇S505

PERFORM MATCHING BETWEEN RIGHT PATCH AND LEFT PATCH GROUP ⌇S506

CALCULATE DISTANCE D FROM LEFT PATCH HAVING MAXIMUM SCORE ⌇S507

DISTANCE D ≤ 0? ⌇S508 — NO

YES

HAVE ALL N RIGHT PATCHES BEEN EVALUATED? ⌇S510 — NO

UPDATE PATCH NUMBER ⌇S512

YES

DETERMINE THAT IMPERSONATION HAS OCCURRED ⌇S509

DETERMINE THAT IMPERSONATION HAS NOT OCCURRED ⌇S511

END

# FIG.25

# FIG.26

START

↓ S501

ACQUIRE LEFT IMAGE AND RIGHT IMAGE

↓ S502

ACQUIRE BACKGROUND LEFT IMAGE AND BACKGROUND RIGHT IMAGE

↓ S503

DIVIDE BACKGROUND RIGHT IMAGE INTO N RIGHT PATCHES

↓ S801

EXECUTE EDGE FILTER PROCESSING ON N RIGHT PATCHES

↓ S504

INITIALIZE PATCH NUMBER

↓ S802

DOES EDGE EXIST IN RIGHT PATCH?

— NO

YES ↓ S505

GENERATE LEFT PATCH GROUP FROM BACKGROUND LEFT IMAGE

↓ S506

PERFORM MATCHING BETWEEN RIGHT PATCH AND LEFT PATCH GROUP

↓ S507

CALCULATE DISTANCE D FROM LEFT PATCH HAVING MAXIMUM SCORE

↓ S508

DISTANCE D ≤ 0?  — NO

S901

HAVE ALL N RIGHT PATCHES BEEN EVALUATED FOR EDGE?

NO ↓ S902

UPDATE PATCH NUMBER

YES

S510

HAVE ALL N RIGHT PATCHES BEEN EVALUATED?  — NO → S512 UPDATE PATCH NUMBER

YES ↓ S508 / S509

YES ↓ S509

DETERMINE THAT IMPERSONATION HAS OCCURRED

S511

DETERMINE THAT IMPERSONATION HAS NOT OCCURRED

END

# FIG.27

START

ACQUIRE LEFT IMAGE AND
RIGHT IMAGE — S501

ACQUIRE BACKGROUND
LEFT IMAGE AND BACKGROUND
RIGHT IMAGE — S502

IS
THERE IMPERSONATION
MATERIAL? — S1001

YES → (to S509)

NO

DIVIDE BACKGROUND RIGHT IMAGE
INTO N RIGHT PATCHES — S503

INITIALIZE PATCH NUMBER — S504

GENERATE LEFT PATCH GROUP
FROM BACKGROUND LEFT IMAGE — S505

PERFORM MATCHING BETWEEN
RIGHT PATCH AND LEFT PATCH
GROUP — S506

CALCULATE DISTANCE D FROM
LEFT PATCH HAVING MAXIMUM
SCORE — S507

DISTANCE D ≤ 0? — S508

NO →

HAVE
ALL N RIGHT
PATCHES BEEN
EVALUAT-
ED? — S510

NO → UPDATE PATCH NUMBER — S512

YES

DETERMINE THAT
IMPERSONATION HAS
NOT OCCURRED — S511

YES

DETERMINE THAT IMPERSONATION
HAS OCCURRED — S509

END

# FIG.28

```
                    ┌─────────────┐
                    │    START    │
                    └──────┬──────┘
                           │
                           ▼          ⌐S401
                    ┌──────────────────────┐
                    │  ACQUIRE LEFT IMAGE   │
                    └──────────┬───────────┘
                               │
                               ▼       ⌐S402
                         ◇───────────◇       NO        ⌐S403
                        ╱ IS PALM REGION ╲──────────┐  ┌──────────────────────┐
                        ╲   INCLUDED?    ╱          └─▶│ ISSUE INSTRUCTION TO  │
                         ◇───────────◇               │      HOLD HAND        │
                               │ YES                 └──────────┬───────────┘
                               ▼       ⌐S404                    │
                         ◇───────────◇       NO        ⌐S405    │
                        ╱ IS BACKGROUND ╲──────────┐  ┌──────────────────────┐
                        ╲REGION INCLUDED?╱          └─▶│ ISSUE INSTRUCTION TO  │
                         ◇───────────◇               │     RELEASE HAND      │
                               │ YES                 └──────────┬───────────┘
                               ▼       ⌐S406                    │
                         ◇───────────◇       YES       ⌐S407    │
                        ╱ IS BACKGROUND ╲──────────┐  ┌──────────────────────┐
                        ╲   UNIFORM?    ╱          └─▶│ ISSUE INSTRUCTION TO  │
                         ◇───────────◇               │  CHANGE BACKGROUND    │
                               │ NO                  └──────────┬───────────┘
                               ▼       ⌐S1101                   │
                    ┌──────────────────────┐                    │
                    │ FOCUS ON PALM REGION  │                    │
                    └──────────┬───────────┘                    │
                               │       ⌐S408                    │
                               ▼                                │
                    ┌──────────────────────┐                    │
                    │ ACQUIRE LEFT IMAGE AND│                    │
                    │     RIGHT IMAGE       │                    │
                    └──────────┬───────────┘                    │
                               │                                │
                               ▼                                │
                    ┌─────────────┐                             │
                    │     END     │                             │
                    └─────────────┘                             │
```

# FIG.29

```
              ┌─────────────┐
              │    START    │
              └──────┬──────┘
                     │         ⌒S501
         ┌───────────▼───────────┐
         │ ACQUIRE LEFT IMAGE AND│
         │      RIGHT IMAGE      │
         └───────────┬───────────┘
                     │         ⌒S502
         ┌───────────▼───────────┐
         │   ACQUIRE BACKGROUND  │
         │ LEFT IMAGE AND BACKGROUND│
         │      RIGHT IMAGE      │
         └───────────┬───────────┘
                     │         ⌒S503
         ┌───────────▼───────────┐
         │DIVIDE BACKGROUND RIGHT IMAGE│
         │   INTO N RIGHT PATCHES│
         └───────────┬───────────┘
                     │         ⌒S504
         ┌───────────▼───────────┐
         │ INITIALIZE PATCH NUMBER│
         └───────────┬───────────┘
                     │         ⌒S505
         ┌───────────▼───────────┐
         │ GENERATE LEFT PATCH GROUP│
         │ FROM BACKGROUND LEFT IMAGE│
         └───────────┬───────────┘
                     │         ⌒S506
         ┌───────────▼───────────┐
         │PERFORM MATCHING BETWEEN│
         │ RIGHT PATCH AND LEFT PATCH│
         │        GROUP         │
         └───────────┬───────────┘
                     │         ⌒S507
         ┌───────────▼───────────┐
         │ CALCULATE DISTANCE D FROM│
         │ LEFT PATCH HAVING MAXIMUM│
         │        SCORE         │
         └───────────┬───────────┘
```

- S501 ACQUIRE LEFT IMAGE AND RIGHT IMAGE
- S502 ACQUIRE BACKGROUND LEFT IMAGE AND BACKGROUND RIGHT IMAGE
- S503 DIVIDE BACKGROUND RIGHT IMAGE INTO N RIGHT PATCHES
- S504 INITIALIZE PATCH NUMBER
- S505 GENERATE LEFT PATCH GROUP FROM BACKGROUND LEFT IMAGE
- S506 PERFORM MATCHING BETWEEN RIGHT PATCH AND LEFT PATCH GROUP
- S507 CALCULATE DISTANCE D FROM LEFT PATCH HAVING MAXIMUM SCORE
- S1201 DISTANCE D = 0? — NO / YES
- S510 HAVE ALL N RIGHT PATCHES BEEN EVALUATED? — NO / YES
- S512 UPDATE PATCH NUMBER
- S509 DETERMINE THAT IMPERSONATION HAS OCCURRED
- S511 DETERMINE THAT IMPERSONATION HAS NOT OCCURRED
- END

# FIG.30

## INTERNATIONAL SEARCH REPORT

| International application No. |
|---|
| **PCT/JP2023/004880** |

### A. CLASSIFICATION OF SUBJECT MATTER

*G06V 40/40*(2022.01)i; *A61B 5/1171*(2016.01)i
FI: G06V40/40; A61B5/1171

According to International Patent Classification (IPC) or to both national classification and IPC

### B. FIELDS SEARCHED

Minimum documentation searched (classification system followed by classification symbols)

G06V40/40; A61B5/1171

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y | WO 2009/110323 A1 (NEC CORP) 11 September 2009 (2009-09-11) claims 1-15, paragraphs [0026]-[0027] | 1-2, 5-15 |
| A | | 3-4 |
| Y | JP 2018-173731 A (MITSUMI ELECTRIC CO LTD) 08 November 2018 (2018-11-08) claims 1-6, paragraphs [0023], [0030]-[0037], [0041] | 1-2, 5-15 |
| A | | 3-4 |
| Y | JP 2021-9609 A (PANASONIC INTELLECTUAL PROPERTY MANAGEMENT CO., LTD.) 28 January 2021 (2021-01-28) paragraph [0023] | 5–6 |
| Y | JP 2021-131737 A (HITACHI LTD) 09 September 2021 (2021-09-09) paragraphs [0020]-[0022], [0074], fig. 2 | 8-10 |
| A | JP 2007-241402 A (SHARP CORP) 20 September 2007 (2007-09-20) claims 5-11, 13-14, paragraphs [0034]-[0039], fig. 1, 3, 5 | 1-15 |

☑ Further documents are listed in the continuation of Box C.   ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **22 March 2023** | **04 April 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT

| | International application No. |
|---|---|
| | **PCT/JP2023/004880** |

**C. DOCUMENTS CONSIDERED TO BE RELEVANT**

| Category* | Citation of document, with indication, where appropriate, of the relevant passages | Relevant to claim No. |
|---|---|---|
| A | WO 2021/074032 A1 (ASSA ABLOY AB) 22 April 2021 (2021-04-22) claims 1-20, paragraph [0050] | 1-15 |

Form PCT/ISA/210 (second sheet) (January 2015)

**INTERNATIONAL SEARCH REPORT**
Information on patent family members

| | International application No. |
|---|---|
| | **PCT/JP2023/004880** |

| Patent document cited in search report | | | Publication date (day/month/year) | Patent family member(s) | | | Publication date (day/month/year) |
|---|---|---|---|---|---|---|---|
| WO | 2009/110323 | A1 | 11 September 2009 | (Family: none) | | | |
| JP | 2018-173731 | A | 08 November 2018 | US<br>claims 1-6, paragraphs [0028],<br>[0035]-[0042], [0046]<br>WO<br>CN | 2020/0026906<br><br><br>2018/181819<br>110678871 | A1<br><br><br>A1<br>A | |
| JP | 2021-9609 | A | 28 January 2021 | US<br>paragraph [0033]<br>WO<br>EP<br>CN | 2022/0245979<br><br>2021/002079<br>3996057<br>114026618 | A1<br><br>A1<br>A1<br>A | |
| JP | 2021-131737 | A | 09 September 2021 | WO<br>paragraphs [0021]-[0023],<br>[0075], fig. 2 | 2021/166289 | A1 | |
| JP | 2007-241402 | A | 20 September 2007 | (Family: none) | | | |
| WO | 2021/074032 | A1 | 22 April 2021 | US<br>claims 1-20, paragraph [0050]<br>KR 10-2022-0070052<br>CN<br>JP | 2021/0110185<br><br><br>114730507<br>2022-546873 | A1<br><br>A<br>A<br>A | |

Form PCT/ISA/210 (patent family annex) (January 2015)

**EP 4 495 900 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2018173731 A